# EUROPEAN PATENT APPLICATION

(11) **EP 3 266 466 A1**
(43) Date of publication of application: **10.01.2018**
(21) Application number: 17163164.1
(22) Date of filing: 18.12.2013
(51) Int. Cl.: A61K 39/395, A61P 35/00, C07K 16/22

(54) **LIQUID ANTIBODY FORMULATION WITH IMPROVED AGGREGATION PROPERTIES**

(30) Priority: 20.12.2012 US 201261740193 P
(62) Divisional of application: 13866178.0
(71) Applicant: Medimmune, LLC, Gaithersburg, Maryland 20878 (US)
(72) Inventor: MALIK, Priyanka, Gaithersburg, MD 20878 (US); SHAH, Ambarish, Gaithersburg, MD 20878 (US); TUESCA, Anthony, Gaithersburg, MD 20878 (US)
(74) Representative: AstraZeneca

(57) **Abstract**

Disclosed here are methods, systems and containers liquid antibody formulations having an improved aggregation profile. In one embodiment, the antibody is a fully monoclonal antibody that binds to Angiopoietin-2.

## Description

### Background

Therapeutic proteins such as antibodies are a rapidly growing class of pharmaceuticals, with over 20 antibody therapeutics having been approved by the U.S. Food and Drug Administration (FDA). Currently many antibody therapeutics are provided as lyophilized formulations. However, lyophilized formulations have limitations, for example, the increased cost associated with a lyophilization manufacturing process, the fact that a lyophilized formulation has to be reconstituted prior to administration and that the reconstituted solutions must be used within a certain time period, which can result in costly waste. Thus, there is a desire to develop liquid antibody formulations at a concentration comparable to or higher than reconstituted lyophilized formulations.

An antibody-containing liquid formulation generally includes a large amount of antibody per administration (e.g., about 100 mg to about 200 mg) and the amount of injection solution can be limited, for example, in a subcutaneous injection. As such, in many cases, high concentration formulations (e.g., about 100 mg/mL) are necessary. However, antibodies in liquid formulations tend to aggregate when stored at high concentrations.

The term "aggregate" can refer to a wide variety of aggregates encountered in biopharmaceutical samples, which can range in size and characteristics (e.g., soluble or insoluble, covalent or noncovalent, reversible or irreversible). Protein aggregation can occur at any step during the manufacturing process (cell culture, purification and formulation), storage, distribution and handling of products and can result from a variety of stresses, for example, agitation and exposure to extremes of pH, temperature, ionic strength, or various interfaces.

For protein therapeutics, the presence of aggregates is typically undesirable because the aggregates may lead to loss of activity, an immunogenic reaction or may cause adverse events on administration, such as obstruction of vessels. In fact, there are guidelines and limitations on the number of particles (≥10 µm and ≥25 µm in size) that may be present in pharmaceutical preparations.

Methods are known for detecting, quantifying and characterizing protein aggregates, including, for example, dynamic light scattering, analytical ultracentrifugation, extrinsic fluorescent dyes, UV/VIS, SDS-PAGE, asymmetrical flow-field flow fractionation, turbidimetry/light obscuration, FTIR, HP-SEC, and multi-angle laser light scattering.

Consequently, there is a desire for improved liquid antibody formulations that have reduced incidence of insoluble aggregation.

### Summary

Described herein is a liquid antibody formulation having an improved aggregation profile. In particular, a liquid antibody formulation having reduced insoluble aggregation is provided. The liquid formulation includes: a fully human monoclonal antibody; and a pharmaceutically acceptable carrier, wherein no more than about 5% of the antibody in the formulation forms an aggregate, in particular, wherein no more than about 5% of the antibody in the formulation forms an insoluble aggregate.

In an embodiment, the invention relates to a method of improving the aggregation profile of a liquid antibody formulation and to liquid antibody formulations prepared by this method. The method includes steps of dispensing a quantity of liquid antibody formulation into one or more non-glass containers; and storing the liquid antibody formulation in the container for a desired period of time, wherein no more than about 5% of the antibody in the formulation forms an aggregate during the storage time period.

In an embodiment, the liquid antibody formulation is stored at a temperature between about 20 °C to about 25 °C for a period of between about 6 months and about 12 months; at a temperature between about 2°C to about 8°C for a period of up to about 12 months; or at a temperature between about 38°C and 42°C for up to about 6 months. In an embodiment, the non-glass container is constructed from a material that includes polymer selected from polypropylene (PP), polyethylene terephthalate (PETG), high-density polyethylene (HDPE), polyethylene terephthalate (PET), polypentafluorostyrene (PFS), polycarbonate, polyvinyl chloride (PVC), polycyclopentane (CZ®), cyclic olefin copolymer (COC), and combinations or copolymers thereof.

In an embodiment, the fill volume in the container can be varied to change the amount of air in the headspace of the container. In some embodiments, the vial can be filled completely with liquid leaving very little air in the headspace, this may result in an air:liquid ratio of 0:1. In other embodiments, the fill volume can be reduced, for example, to an air:liquid ratio of 9:1. In an embodiment, the quantity of liquid antibody formulation is dispensed into the container to provide an air to liquid ratio of volumeₐᵢᵣ to volume_{liquid} of less than or equal to 0.05. In an embodiment, the container is closed with a container closure capable of reducing exposure of the liquid formulation to air polymeric container types including polypropylene (PP), polyethylene terephthalate (PETG), high-density polyethylene (HDPE), polyethylene terephthalate (PET), polypentafluorostyrene (PFS), polycarbonate, polyvinyl chloride (PVC), polycyclopentane (CZ®), cyclic olefin copolymer (COC), and combinations or copolymers thereof.

In an embodiment, the liquid formulation includes between about 1 mM and about 100 mM non-zwitterionic buffer selected from phosphate, tris, citrate, succinate, acetate, or a combination thereof.

In an embodiment, the liquid formulation includes a fully human monoclonal antibody that binds to angiopoietin-2 and a pharmaceutically acceptable carrier. In one embodiment, the fully human monoclonal antibody binds to angiopoietin-2. In another embodiment, the antibody that binds to angiopoietin-2 is a fully human monoclonal antibody comprising a variable light chain selected from: a light chain sequence comprising SEQ ID NO: 12; a light chain sequence comprising SEQ ID NO: 20; and a light chain sequence comprising SEQ ID NO: 32. In another embodiment, the antibody is a fully human monoclonal antibody comprising a variable heavy chain selected from: a heavy chain sequence comprising SEQ ID NO: 10; a heavy chain sequence comprising SEQ ID NO: 18; and a heavy chain sequence comprising SEQ ID NO: 30. In another embodiment, the antibody is a fully human monoclonal antibody comprising a variable light chain and a variable heavy chain member selected from: a light chain sequence comprising SEQ ID NO: 12 and a heavy chain sequence comprising SEQ ID NO: 10; a light chain sequence comprising SEQ ID NO: 20 and a heavy chain sequence comprising SEQ ID NO: 18; and a light chain sequence comprising SEQ ID NO: 32 and a heavy chain sequence comprising SEQ ID NO: 30. In another embodiment, the antibody is a fully human monoclonal antibody selected from an antibody comprising heavy chain CDR1, CDR2, CDR3 of SEQ ID NO: 10 and light chain CDR1, CDR2, CDR3 of SEQ ID NO: 12; heavy chain CDR1, CDR2, CDR3 of SEQ ID NO: 18 and light chain CDR1, CDR2, CDR3 of SEQ ID NO: 20; and heavy chain CDR1, CDR2, CDR3 of SEQ ID NO: 30 and light chain CDR1, CDR2, CDR3 of SEQ ID NO: 32.

In another embodiment, the antibodies comprise a variable light chain amino acid sequence selected from the group consisting of MEDI1 (SEQ ID No.:33), MEDI2 (SEQ ID No.:34), MEDI3 (SEQ ID No.:35), MEDI6 (SEQ ID No.:38) and MEDI4 (SEQ ID No.:36). In another embodiment, the antibodies comprise the heavy chain variable amino acid sequence MEDI5 (SEQ ID No.:37). In another embodiment, the antibodies comprise a variable light chain amino acid sequence selected from the group consisting of MEDI1 (SEQ ID No.:33), MEDI2 (SEQ ID No.:34), MEDI3 (SEQ ID No.:35), MEDI6 (SEQ ID No.:38) and MEDI4 (SEQ ID No.:36) as well as a heavy chain variable sequence defined as MEDI5 (SEQ ID No.:37). In an embodiment, the antibody that binds to angiopoietin-2 is mAb 3.19.3 (ATCC Accession Number PTA-7260); mAb 3.3.2 (ATCC Accession Number PTA-7258); or mAb 5.88.3 (ATCC Accession Number PTA-7259).

In one embodiment, the liquid formulation has a pH between about 5.0 and about 8.0.

In one embodiment, the aggregate formed by no more than about 5% of the antibody in the formulation comprises visible particles, subvisible particles, precipitates, fibrils, or a combination thereof. In an embodiment, the aggregate comprises sub-visible particles greater than at least about 2 µm and up to about 200 µm. In another embodiment, the aggregate comprises visible particles greater than at least about 200 µm.

In one embodiment, the pharmaceutically acceptable carrier comprises distilled water.

The invention described herein also provides an article of manufacture that includes one or more non-glass primary packaging components comprising one or more containers containing a liquid antibody formulation having an improved aggregation profile, wherein no more than about 5% of the antibody in the formulation forms an aggregate. In one embodiment, the liquid formulation includes a fully human monoclonal antibody that binds to angiopoietin-2 in a pharmaceutically acceptable carrier. In one embodiment, the one or more containers are non-glass containers selected from an ampule, vial, bottle and combinations thereof. In one embodiment, the one or more non-glass containers are constructed from a material that includes polymer selected from polypropylene (PP), polyethylene terephthalate (PETG), high-density polyethylene (HDPE), polypentafluorostyrene (PFS), polycarbonate, polyvinyl chloride (PVC), crystal zenith (CZ®)cyclic olefin copolymer (COC), and combinations or copolymers thereof. In a more specific embodiment, the non-glass container is constructed from material that includes polypropylene. In one embodiment, the container contains air and the liquid formulation in a ratio of volumeₐᵢᵣ to volume_{liquid} of less than or equal to 0.05.

This summary is an overview of some of the teachings of the present application and is not intended to be an exclusive or exhaustive treatment of the present subject matter. Further details are found in the detailed description, examples, and appended claims. Other aspects of the invention will be apparent to persons skilled in the art upon reading and understanding the following detailed description and examples, and viewing the drawings that form a part thereof, each of which is not to be taken in a limiting sense. The scope of the present invention is defined by the appended claims and their legal equivalents.

### Brief Description of the Drawings

Figure 1 is a table showing the visible particle (VP) counts observed over an 8 day period for various fill volumes in a 5 cc container.
Figure 2 is a graph showing the sub visible particle (SVP) counts observed over an 8 day period for various fill volumes in a 5 cc container.
Figure 3 is a photograph showing the container types used in the study.
Figure 4 is an illustration and table showing the surface area (SA) of liquid exposed container to volume (V) of liquid ratios for a variety of container types.
Figure 5 is a graph showing the sub visible particle (SVP) (> 10 µm) count in various container types over a 70 day period.
Figure 6 is a graph showing the sub visible particle (SVP) (> 10 µm) counts for various container/bead combinations over a 21 day period.
Figure 7 is graph showing the sub visible particle (SVP) counts/mL for nitrogen purging study.
Figure 8 is a graph showing sub visible particle (SVP) counts/mL for agitation study.
Figure 9 is a photograph showing sub visible particles for agitation study.

While the invention is susceptible to various modifications and alternative forms, specifics thereof have been shown by way of example and drawings, and will be described in detail. It should be understood, however, that the invention is not limited to the particular embodiments described. On the contrary, the intention is to second modifications, equivalents, and alternatives falling within the spirit and scope of the invention.

### Detailed Description

### Overview

There exists a substantial interest in using therapeutic antibodies for the treatment of a variety of diseases. In particular, there exists substantial interest in liquid formulations of therapeutic antibodies. However, antibodies can exhibit a tendency to aggregate when stored as a liquid formulation, particularly when a high concentration of antibody is included in the formulation. Described herein is a system and method for reducing the formation of aggregations in a liquid antibody formulation.

### Definitions

As used herein, the term "about" is used to modify, for example, the quantity of an ingredient in a composition, concentration, volume, process temperature, process time, yield, flow rate, pressure, and ranges thereof, employed in describing the invention. The term "about" refers to variation in the numerical quantity that can occur, for example, through typical measuring and handling procedures used for making compounds, compositions, concentrates or use formulations; through inadvertent error in these procedures; through differences in the manufacture, source, or purity of starting materials or ingredients used to carry out the methods, and other similar considerations. The term "about" also encompasses amounts that differ due to aging of a formulation with a particular initial concentration or mixture, and amounts that differ due to mixing or processing a formulation with a particular initial concentration or mixture. Where modified by the term "about" the claims appended hereto include such equivalents.

As used herein, the term "aggregate" refers to the association of protein molecules and includes a wide variety of aggregates which can range in size and characteristics, including for example covalent or non-covalent, soluble or insoluble and reversible or irreversible. As used herein, the term "soluble aggregate" refers to aggregates that are not visible to the naked eye as discrete particles and that cannot be removed by a filter with a pore size of 0.22 µm. As used herein, the term "insoluble aggregate" refers to aggregates that can be removed by filtration and may be visible to the unaided eye.

The term "Ang-2" refers to the molecule Angiopoietin-2.

"Binding fragments" of an antibody can be produced by known recombinant DNA techniques, or by enzymatic or chemical cleavage of intact antibodies. Digestion of an antibody with the enzyme papain results in two identical antigen-binding fragments, known also as "Fab" fragments, and a "Fc" fragment, having no antigen-binding activity but having the ability to crystallize. Digestion of antibodies with the enzyme pepsin results in the F(ab')₂ fragment in which the two arms of the antibody molecule remain linked and include two-antigen binding sites. The F(ab')₂ fragment has the ability to crosslink antigen. "Fv" when used herein refers to the minimum fragment of an antibody that retains both antigen-recognition and antigen-binding sites. An antibody other than a "bispecific" or "bifunctional" antibody is understood to have each of its binding sites identical.

The term "epitope" includes any protein determinant capable of specific binding to an immunoglobulin. Epitopic determinants usually consist of chemically active surface groupings of molecules such as amino acids or sugar side chains and may, but not always, have specific three-dimensional structural characteristics, as well as specific charge characteristics. An antibody is said to specifically bind an antigen when the dissociation constant between the antibody and epitope is less than or equal to about 1 µM, less than or equal to about 100 nM or less than or equal to about 10 nM.

The term "isolated protein" refers to a protein that has been isolated from its naturally occurring environment. Such proteins may be derived from genomic DNA, cDNA, recombinant DNA, recombinant RNA, or synthetic origin or some combination thereof, which by virtue of its origin, or source of derivation, the "isolated protein" (1) is not associated with proteins found in nature, (2) is free of other proteins from the same source, e.g. free of murine proteins, (3) is expressed by a cell from a different species, or (4) does not occur in nature.

The term "mAb" refers to monoclonal antibody.

The term "naturally-occurring" as used herein as applied to an object refers to the fact that an object can be found in nature. For example, a polypeptide or polynucleotide sequence that is present in an organism (including viruses) that can be isolated from a source in nature and which has not been intentionally modified by man in the laboratory or otherwise is naturally-occurring.

The term "neutralizing" when referring to an antibody relates to the ability of an antibody to eliminate, or significantly reduce, the activity of a target antigen. Accordingly, a "neutralizing" anti-Ang-2 antibody is capable of eliminating or significantly reducing the activity of Ang-2. A neutralizing Ang-2 antibody may, for example, act by blocking the binding of Ang-2 to its receptor Tie2. By blocking this binding, the Tie2 mediated signal transduction can be significantly, or completely, eliminated. In one embodiment, a neutralizing antibody against Ang-2 inhibits angiogenesis.

The term "pharmaceutical agent or drug" as used herein refers to a chemical compound or composition capable of inducing a desired therapeutic effect when properly administered to a patient.

The phrase "pharmaceutically acceptable" means approved by a regulatory agency of the Federal or a state government, or listed in the U.S. Pharmacopeia, European Pharmacopeia, or other generally recognized pharmacopeia for use in animals, in particular, for use in humans.

The term "polypeptide" is used herein as a generic term to refer to native protein, fragments, or analogs of a polypeptide sequence. Hence, native protein, fragments, and analogs are species of the polypeptide genus. In one embodiment, polypeptide refers to the human heavy chain immunoglobulin molecules and the human kappa light chain immunoglobulin molecules, as well as antibody molecules formed by combinations including the heavy chain immunoglobulin molecules with light chain immunoglobulin molecules, such as the kappa or lambda light chain immunoglobulin molecules, and vice versa, as well as fragments and analogs thereof.

As used herein, the terms "prevent," "preventing," and "prevention" refer to the inhibition of the development or onset of disease or disorder, for example, a disease or disorder associated with or characterized by aberrant expression and/or activity of ang-2.

As used herein, the terms "prophylactic agent" and "prophylactic agents" refer to any agent(s) which can be used in the prevention of the onset, recurrence or development of a disease or disorder, for example, a disease or disorder associated with or characterized by aberrant expression and/or activity of ang-2.

As used herein, the term "prophylactically effective amount" refers to the amount of a therapy (e.g., prophylactic agent) which is sufficient to result in the prevention of the development, recurrence, or onset of a disease or disorder, for example, a disease or disorder associated with or characterized by aberrant expression and/or activity of an ang-2.

A used herein, a "protocol" includes dosing schedules and dosing regimens. The protocols herein are methods of use and include prophylactic and therapeutic protocols.

The terms "stability" and "stable" as used herein in-the context of a liquid formulation comprising an antibody (including antibody fragment thereof) that immunospecifically binds to an antigen of interest (e.g., Ang-2) refer to the resistance of the antibody (including antibody fragment thereof) in the liquid antibody formulation to form an aggregate, when prepared under the manufacture, preparation, transportation and storage conditions of the invention. The "stable" formulations of the invention demonstrate an improved aggregation profile under given manufacture, preparation, transportation and storage conditions as compared to a reference formulation.

As used herein, the terms "subject" and "patient" are used interchangeably. As used herein, the terms "subject" and "subjects" refer to an animal, preferably a mammal including a non-primate (e.g., a cow, pig, horse, cat, dog, rat, and mouse) and a primate (e.g., a monkey, such as a cynomolgous monkey, chimpanzee, and a human), and more specifically a human.

As used herein, the terms "therapeutic agent" and "therapeutic agents" refer to any agent(s) which can be used in the prevention, treatment and/or management of a disease or disorder, for example, a disease or disorder associated with or characterized by aberrant expression and/or activity of ang-2. In certain other embodiments, the term "therapeutic agent" refers an agent other than an antibody that immunospecifically binds to ang-2.

As used herein, the term "therapeutically effective amount" refers to the amount of a therapy that is sufficient to reduce the severity of a disease or disorder

As used herein, the terms "treat," "treatment," and "treating" refer to the reduction or amelioration of the progression, severity, and/or duration of a disease or disorder, for example, a disease or disorder associated with or characterized by aberrant expression and/or activity of ang-2.

Unless otherwise defined, scientific and technical terms used herein shall have the meanings that are commonly understood by those of ordinary skill in the art. Further, unless otherwise required by context, singular terms shall include pluralities and plural terms shall include the singular. Generally, nomenclatures utilized in connection with, and techniques of, cell and tissue culture, molecular biology, and protein and oligo- or polynucleotide chemistry and hybridization described herein are those well known and commonly used in the art.

Standard techniques are used for recombinant DNA, oligonucleotide synthesis, and tissue culture and transformation (e.g., electroporation, lipofection). Enzymatic reactions and purification techniques are performed according to manufacturer's specifications or as commonly accomplished in the art or as described herein. The techniques and procedures are generally performed according to conventional methods well known in the art and as described in various general and more specific references that are cited and discussed throughout the present specification. See e.g., Sambrook et al. Molecular Cloning: A Laboratory Manual (3rd ed., Cold Spring Harbor Laboratory Press, Cold Spring Harbor, N.Y. (2001)), which is incorporated herein by reference. The nomenclatures utilized in connection with, and the laboratory procedures and techniques of, analytical chemistry, synthetic organic chemistry, and medicinal and pharmaceutical chemistry described herein are those well known and commonly used in the art. Standard techniques are used for chemical syntheses, chemical analyses, pharmaceutical preparation, formulation, and delivery, and treatment of patients.

### Antibody Formulation

The invention is directed towards a stable liquid antibody formulation which exhibits an improved aggregation profile (e.g., little to no aggregation) during storage. In one embodiment, such antibody formulations are homogeneous. In one embodiment, the formulations of the invention are sterile. The formulations of the present invention include a pharmaceutically acceptable carrier and a therapeutic or prophylactic antibody of interest (including antibody fragments thereof). In one embodiment, the therapeutic antibody is an antibody that immunospecifically binds to angiopoietin-2. In one embodiment, the pharmaceutically acceptable carrier is an aqueous carrier, in a more specific embodiment, the aqueous carrier includes water. In a more specific embodiment, the water is distilled.

Methods for developing a liquid formulation are known and include, for example, selecting the antibody, the optimum solution pH, buffer type and concentration, evaluating the effect of various excipients on the stability, and optimizing the concentration of the screened excipients using an I-optimal experimental design (Statistics for Experimenters: An Introduction to Design, Data Analysis, and Model Building, Box, George E. P. et al., John Wiley and Sons, Inc., 1978).

An effective amount of antibody to be employed therapeutically will depend, for example, upon the therapeutic objectives, the route of administration, and the condition of the patient. Other considerations include severity and type of disease, body weight, sex, diet, time and route of administration, other medications and other relevant clinical factors. It is expected that the therapist titer the dosage and modify the route of administration as required to obtain the optimal therapeutic effect. Typically, the clinician will administer antibody until a dosage is reached that achieves the desired effect. The progress of this therapy is easily monitored by conventional assays or by the assays described herein.

The pH of the formulation is generally not equal to the isoelectric point of the therapeutic antibody (or antibody fragment) used in the formulation. In general, the pH of the formulation ranges from between about 5.0 and about 8.0. Examples of buffers that can be used to prepare the liquid antibody formulations of the invention include, non-zwitterionic buffers. The non-zwitterionic buffers used in the methods and formulations of the invention may be, organic acid buffers or buffers such as succinate (such as sodium succinate), gluconate, histidine, citrate (such as sodium citrate, for example sodium citrate/citric acid), phosphate, tris, acetate, or a combination thereof.

In one embodiment, the excipient includes a saccharide (e.g., a sugar).

In another embodiment, the excipient includes a surfactant. While not wishing to be bound by theory, it is believed that surfactants may reduce aggregation by a competitive interaction at the air/water interface of the liquid formulation which reduces exposure of the antibody to air. In a more specific embodiment, the surfactant is a nonionic surfactant such as polysorbate (e.g., polysorbates 20 and 80, such as Tween™-20 or Tween™ -80) or poloxamer (e.g., poloxamer 188). In general, the surfactant is added in an amount sufficient to reduce aggregation of the formulated antibody, including, for example, reducing visible and subvisible particles in the formulation. In one embodiment, the surfactant is present in the formulation at a concentration of at least about 0.001 % and up to about 1% of the formulation. In some embodiments, the surfactant is present in the formulation at a concentration from between about 0% to about 0.1 %, about 0.01 % to about 0.1 %, or about 0.01 % to about 0.05% of the formulation. In a specific embodiment, the surfactant is Tween-20 or Tween-80, and is present in the formulation at a concentration of at least about 0.001%, 0.005%, 0.01%, 0.02%, 0.05%, or 0.08%, and up to about 0.1%, 0.5%, or 1% of the formulation.

In one embodiment, the formulation includes a suspending agent, including, but not limited to, methyl cellulose, polysorbate 80, hydroxyethyl cellulose, gum arabic, powdered tragacanth, sodium carboxymethylcellulose and polyoxyethylene sorbitan monolaurate, and combinations thereof.

In another embodiment, the formulation includes a solubilizing agent, including, but not limited to, polyoxyethylene hydrogenated castor oil, polysorbate 80, nicotinamide, polyoxyethylene sorbitan monolaurate, macrogol and castor oil fatty acid ethyl ester, and combinations thereof.

In one embodiment, the viscosity of the liquid antibody formulation of the invention is between about 2 mPas to about 15 mPas, about 4 mPas to about 10 mPas. Methods for determining viscosity of liquid formulations are known, and include, for example, use of a rotation viscometer method using a cone-plate type viscometer, in accordance with 2.53 Viscosity Determination/General Tests, the Japanese Pharmacopoeia, 15th edition.

In one embodiment, the liquid antibody formulation of the invention is sterilized by sterile filtration, for example using a 0.2 micron or a 0.22 micron filter.

The liquid antibody formulation of the invention can be prepared as unit dosage forms. For example, a unit dosage per vial may contain at least about 1 mL and up to about 20 mL of the liquid antibody formulation of the invention. In some embodiments, the unit dosage may contain at least about 1 mL, at least about 2 mL, at least about 3 mL, at least about 4 mL, at least about 5 mL, at least about 6 mL, at least about 7 mL, at least about 8 mL, at least about 9 mL, up to about 10 mL, up to about 15 mL, or up to about 20 mL formulation.

In one embodiment, the formulation includes a sustained-release preparation that includes semipermeable matrices of solid hydrophobic polymers containing the antibody, which matrices are in the form of shaped articles, films or microcapsules. Examples of sustained-release matrices include polyesters, hydrogels (e.g., poly(2-hydroxyethyl-methacrylate) as described by Langer et al., J. Biomed Mater. Res., (1981) 15:167-277 and Langer, Chem. Tech., (1982) 12:98-105, or poly(vinylalcohol)), polylactides (U.S. Pat. No. 3,773,919, EP 58,481), copolymers of L-glutamic acid and gamma ethyl-L-glutamate (Sidman et al., Biopolymers, (1983) 22:547-556), non-degradable ethylene-vinyl acetate (Langer et al., supra), degradable lactic acid-glycolic acid copolymers such as the LUPRON Depot™ (injectable microspheres composed of lactic acid-glycolic acid copolymer and leuprolide acetate), and poly-D-(-)-3-hydroxybutyric acid (EP 133,988).

### Aggregation

As used herein, the term "improved aggregation profile" refers to a liquid antibody formulation that has low to undetectable levels of antibody aggregation during manufacture, preparation, transportation, and/or storage. In another embodiment, the formulation has low to undetectable levels of insoluble antibody aggregation. The term aggregation refers to the self-association of two or more antibody molecules that can result in the formation of visible or sub-visible particles, precipitates or fibrils. As used herein, the term "visible particle" is used to refer to antibody aggregates greater than at least about 200 µm, or about 200 µm, or up to about 200 µm or about 300 µm. The term "sub-visible particle" is used to refer to antibody aggregates greater than about 2 µm, about 5 µm, about 10 µm, about 25 µm, about 50 µm, about 75 µm, about 100 µm, and up to about 200 µm. The phrase "low to undetectable levels of aggregation" refers to samples in which no more than about 5% of the antibody or, no more than about 4% of the antibody or, no more than about 3% of the antibody or, no more than about 2% of the antibody or, no more than about 1% of the antibody, or no more than about 0.5% of the antibody (including antibody fragment thereof) in the liquid antibody formulation of the invention forms an aggregate as measured by a known method for determining aggregation, after the storage for a defined period of time, for example, the time periods set forth below. Methods for determining aggregation are known in the art and include, for example, high performance size exclusion chromatography (HPSEC), static light scattering (SLS), Fourier Transform Infrared Spectroscopy (FTIR), circular dichroism (CD), urea-induced protein unfolding techniques, tangential flow filtration (TFF), intrinsic tryptophan fluorescence, differential scanning calorimetry (DSC), and/or 1-anilino-8-naphthalene-sulfonic acid (ANS) protein binding techniques.

In one embodiment, the liquid antibody formulation of the invention maintains an improved aggregation profile upon storage. In another embodiment, the liquid antibody formulation of the invention has a reduced incidence of insoluble aggregation. In one embodiment, the liquid antibody formulation of the invention maintains an improved aggregation profile when stored for extended periods of time at room temperature (between about 20 °C to about 25 °C) or at reduced temperatures (below about 10°C, between about 2°C to about 8°C, for example, at 5°C), for up to about 6 months, 1 year, 2 years, 3 years or 5 years. In another embodiment, the liquid antibody formulation of the invention maintains an improved aggregation profile when stored at elevated temperatures, such as between about 38°C and 42°C, in particular at 40°C for a period of time, such as up to about 1 week, 2 weeks, 3 weeks, 1 month, 2 months, 3 months, 6 months, 9 months, or 1 year. In a more particular embodiment, an antibody formulation of an antibody that specifically binds Angiopoietin-2 maintains an improved aggregation profile when stored for a period of time, such as up to about 1 week, up to about 2 weeks, up to about 3 weeks, up to about 1 month, up to about 2 months, up to about three months, or up to about 6 months at a temperature of between about 2°C to about 8°C.

Stability of the liquid formulations can also be determined by examining biological activities of the antibody (including antibody fragment thereof) during the prolonged storage under the conditions described above, as assessed by various immunological assays including, for example, enzyme-linked immunosorbent assay (ELISA) and radioimmunoassay to measure the ability of the antibody (including antibody fragment thereof) to immunospecifically bind to an antigen. In one embodiment, the liquid antibody formulations of the present invention retain, after storage for the above-defined periods, more than 80%, more than 85%, more than 90%, more than 95%, more than 98%, more than 99%, or more than 99.5% of the initial biological activities of the formulation prior to the storage, i.e., compared to a reference antibody representing the antibody prior to storage.

### Anti-Angiopoietin-2 antibodies

In an embodiment, the liquid antibody formulation of the invention comprises an antibody that specifically binds to Angiopoietin-2 (Ang-2). In one embodiment, the formulation includes monoclonal antibodies that specifically bind Angiopoietin-2 (Ang-2). In a more particular embodiment, the formulation includes fully human monoclonal antibodies directed to Ang-2. In another embodiment, the formulation includes isolated binding fragments of anti-Ang-2 antibodies. In one embodiment, the binding fragments are derived from fully human anti-Ang-2 antibodies. Non-limiting examples of therapeutic or prophylactic antibodies are described in detail in U.S. Patent Publication No. 2006/0246071, entitled "ANTIBODIES DIRECTED TO ANGIOPOIETIN-2 AND USES THEREOF," the disclosure of which is hereby incorporated by reference herein in its entirety.

In one embodiment, the liquid antibody formulation of the invention includes a fully human monoclonal antibody selected from mAb 3.3.2 (ATCC Accession Number PTA-7258), mAb 3.19.3 (ATCC Accession Number PTA-7260) or mAb 5.88.3 (ATCC Accession Number PTA-7259), which specifically bind to Ang-2. In one embodiment, the antibody is a fully human monoclonal antibody mAb 3.19.3. In another embodiment, the antibody binds to the same epitope or epitopes as fully human monoclonal antibody mAb 3.19.3.

In one embodiment, the liquid antibody formulation of the invention includes specific anti-Ang-2 antibodies listed in Table 1. This table reports the identification number of each anti-Ang-2 antibody, along with the SEQ ID number of the corresponding heavy chain and light chain genes. Each antibody has been given an identification number that includes either two or three numbers separated by one or two decimal points. For most of the antibodies, only two identification numbers, separated by one decimal point, are listed.

In one embodiment, the antibody includes a heavy chain amino acid sequence having a complementarity determining region (CDR) of one of the sequences shown in Table 1. It is noted that those of ordinary skill in the art can readily accomplish CDR determinations. See for example, Kabat et al., Sequences of Proteins of Immunological Interest, Fifth Edition, NIH Publication 91-3242, Bethesda Md. (1991), vols. 1-3. In another embodiment, the antibody that binds to Ang-2 and includes a light chain amino acid sequence having a CDR including one of the sequences shown in Table 1. In another embodiment, the antibody is an antibody that binds to Ang-2 and includes a heavy chain amino acid sequence having one or more of the CDR sequences shown in Table 1 and a light chain amino acid sequence having one or more of the CDR sequences shown in Table 1. In one embodiment the antibody cross-competes for binding to Ang-2 with the fully human antibodies of the invention and includes a heavy chain amino acid sequence having one or more of the CDR sequences shown in Table 1 and a light chain amino acid sequence having one or more of the CDR sequences shown in Table 1.

In another embodiment, the antibody binds to the same epitope on Ang-2 as a fully human monoclonal antibody selected from mAb 3.3.2 (ATCC Accession Number PTA-7258), mAb 3.19.3 (ATCC Accession Number PTA-7260) or mAb 5.88.3 (ATCC Accession Number PTA-7259). In one embodiment, the antibody includes a heavy chain amino acid sequence having one or more of the CDR sequences shown in Table 3 and a light chain amino acid sequence having one or more of the CDR sequences shown in Table 2.

Each antibody has been given an identification number that includes either two or three numbers separated by one or two decimal points. For most of the antibodies, only two identification numbers, separated by one decimal point, are listed. However, in some cases, several clones of one antibody were prepared. Although the clones have the identical nucleic acid and amino acid sequences as the parent sequence, they may also be listed separately, with the clone number indicated by the number to the right of a second decimal point. Thus, for example, the nucleic acid and amino acid sequences of antibody 3.3 are identical to the sequences of antibody 3.3.1 and 3.3.2. Similarly, the nucleic acid and amino acid sequences of antibody 3.19 are identical to the sequences of antibody 3.19.1, 3.19.2 and 3.19.3 and the nucleic acid and amino acid sequences of antibody 5.88 are identical to the sequences of antibody 5.88.1, 5.88.2 and 5.88.3.

**Table 1**

| **CDR SEQUENCES OF THE ANTI Ang-2 ANTIBODIES** | | |
|---|---|---|
| **mAb ID No:** | **Sequence** | **SEQ ID NO:** |
| 3.3 | Nucleotide sequence encoding the variable region of the heavy chain | 1 |
| | Amino acid sequence of the variable region of the heavy chain | 2 |
| | Nucleotide sequence encoding the variable region of the light chain | 3 |
| | Amino acid sequence of the variable region of the light chain | 4 |
| 3.3.1 | Nucleotide sequence encoding the variable region of the heavy chain | 5 |
| | Amino acid sequence of the variable region of the heavy chain | 6 |
| | Nucleotide sequence encoding the variable region of the light chain | 7 |
| | Amino acid sequence of the variable region of the light chain | 8 |
| 3.3.2 | Nucleotide sequence encoding the variable region of the heavy chain | 9 |
| | Amino acid sequence of the variable region of the heavy chain | 10 |
| | Nucleotide sequence encoding the variable region of the light chain | 11 |
| | Amino acid sequence of the variable region of the light chain | 12 |
| 3.19.1 | Nucleotide sequence encoding the variable region of the heavy chain | 13 |
| | Amino acid sequence of the variable region of the heavy chain | 14 |
| | Nucleotide sequence encoding the variable region of the light chain | 15 |
| | Amino acid sequence of the variable region of the light chain | 16 |
| 3.19.3 | Nucleotide sequence encoding the variable region of the heavy chain | 17 |
| | Amino acid sequence of the variable region of the heavy chain | 18 |
| | Nucleotide sequence encoding the variable region of the light chain | 19 |
| | Amino acid sequence of the variable region of the light chain | 20 |
| 5.88 | Nucleotide sequence encoding the variable region of the heavy chain | 21 |
| | Amino acid sequence of the variable region of the heavy chain | 22 |
| | Nucleotide sequence encoding the variable region of the light chain | 23 |
| | Amino acid sequence of the variable region of the light chain | 24 |
| 5.88.1 | Nucleotide sequence encoding the variable region of the heavy chain | 25 |
| | Amino acid sequence of the variable region of the heavy chain | 26 |
| | Nucleotide sequence encoding the variable region of the light chain | 27 |
| | Amino acid sequence of the variable region of the light chain | 28 |
| 5.88.3 | Nucleotide sequence encoding the variable region of the heavy chain | 29 |
| | Amino acid sequence of the variable region of the heavy chain | 30 |
| | Nucleotide sequence encoding the variable region of the light chain | 31 |
| | Amino acid sequence of the variable region of the light chain | 32 |

**Table 2: Light Chain Analysis**

| Chath Name | SEQ ID NO | J | FR1 | CDR1 | FR2 | CDR2 | FR3 | CDR3 | J |
|---|---|---|---|---|---|---|---|---|---|
| 5.88.1 | 28 | JK1 | EIVMTQSPATLS VSPGERAILSC | RASQSISSNLA | WYQQKPGQ APRLLIY | GASTRAT | GIPARFSGSGSGTEFT LTISSLQSEDFAVYYC | QQYNYWWT | FGQGKVEIKR |
| 3.3 | 4 | JK1 | EIVMTQSPATLS VSPGERATLSC | RASQTVSSDLA | WYQQKPGQ APRLLIY | GASIRAT | GIPARTSGSGSGTEFT LTISSLQSEDFAVYSC | QQYYNWWT | FGQGKVEIKR |
| 3.19.1 | 16 | JK5 | EIVLTQSPGTLS LSPGERATLSC | RASQSITGSYLA | WYQQKPGQ APRLLIC | GASSWAT | GIPDRFSGSGSGTDFT LTISRLEPEDFAVYYC | QQYSSSPIT | FCQGTRLEIKR |

**Table 3: Heavy Chain Analysis**

| Chain Name | SEQ ID NO: | J | FR1 | CDR1 | FR2 | CDR2 | FR3 | CDR3 | J |
|---|---|---|---|---|---|---|---|---|---|
| 5.88.1 | 26 | JH4B | EVQMVESGGGLVQ PGGSLRLSCAAS | GFTLR SYWMS | WVRQAPG KGLEWVA | NIKEDGSEKY HVDSVKG | RFTISRDNAENSLFLQM SSLRAEDTAVYYCAR | DMEASAG LFDY | WGQGTL VTVSSA |
| 3.3 | 2 | JH4B | | GFTFS SYWMS | WVRQAPG KGLEWVA | NIKQDGSEQY YVDSVKG | RFTISRDNAKNSLYLQM NTLRAEDTAVYYCVR | DQGIAVA GPFDY | WGQGTL VTVSSA |
| 3.19.1 | 14 | JH5B | QVQLVESGGGVVQ PGRSLRLSCAAS | GFTFS NAWMS | WGRQAPG KGLEWVA | VISHDGNNKY YVDSVKG | RFTISRDNSKNTLYLQM NSLRAEDTAVYYCAR | EGIDEWS GLNWFDP | WGQGTL VTVSSA |

Certain modifications can be made to a particular Ang-2 antibody (mAb 3.19.3), which renders the antibody more stable under certain conditions. Stabilized Ang-2 antibodies include antibodies that include a substitution of an amino acid at position 49 (as compared to the light chain variable amino acid sequence of Ang-2 antibody 3.19.3, see SEQ ID No. 16) as defined by the Kabat numbering system (Kabat et al., Sequences of Proteins of Immunological Interest, Fifth Edition, NIH Publication 91-3242, Bethesda MD (1991), vols. 1-3.). The amino acid substitution at position 49 may be selected from Asp, Thr, Asn, and Ala. In particular, altering residue 49 of the light chain can result in much less aggregation. Residue 37 of the heavy chain for mAb 3.19.3 can also be modified to reduce aggregation. The anti Ang-2 antibodies useful in the formulations of the invention may include a substitution of Val substitution at position 37 of the heavy chain (as compared to the heavy chain variable amino acid sequence of Ang-2 antibody 3.19.3, see, SEQ ID No. 14) as defined by the EU numbering system ((Kabat et al., Sequences of Proteins of Immunological Interest, Fifth Edition, NIH Publication 91-3242, Bethesda MD (1991), vols. 1-3.).

The anti Ang-2 antibodies useful in the formulations of the invention may comprise variable light chain acid sequences selected from MEDI1 (SEQ ID No.:33), MEDI2 (SEQ ID No.:34), MEDI3 (SEQ ID No.:35), MEDI6 (SEQ ID NO:38), and MEDI4 (SEQ ID No.:36). The anti Ang-2 antibodies useful in the formulations of the invention may comprise the heavy chain variable sequence of MEDI5 (SEQ ID No.:37). The anti Ang-2 antibodies useful in the formulations of the invention may comprise variable light chain sequences selected from MEDI1 (SEQ ID No.:33), MEDI2 (SEQ ID No.:34), MEDI3 (SEQ ID No.:35), MEDI6 (SEQ ID NO:38), and MEDI4 (SEQ ID No.:36) and further comprise the heavy chain variable sequence of MEDI5 (SEQ ID No.:37). As used herein, an antibody comprising a light chain and a heavy chain may be referred to as a MEDIX/MEDIY wherein X represents the light chain sequence and Y represents the heavy chain sequence.

The anti Ang-2 antibodies useful in the formulations of the invention may comprise variable light chain acid sequences selected from MEDI1 (SEQ ID No.:33), MEDI2 (SEQ ID No.:34), MEDI3 (SEQ ID No.:35), MEDI6 (SEQ ID NO:38), and MEDI4 (SEQ ID No.:36), but having a different amino acid substitution at position 49. The anti-Ang-2 antibodies useful in the formulations of the invention may further comprise the heavy chain variable sequence of MEDI5 (SEQ ID No.:37). the anti Ang-2 antibodies useful in the formulations of the invention may comprise variable light chain sequences selected from MEDI1 (SEQ ID No.:33), MEDI2 (SEQ ID No.:34), MEDI3 (SEQ ID No.:35), MEDI6 (SEQ ID NO:38), and MEDI4 (SEQ ID No.:36), but having a different amino acid substitution at position 49, and further comprise the heavy chain variable sequence of MEDI5 (SEQ ID No.:37).

### Anti-Angiopoietin-2 Antibody Sequences

*3.19.3 light chain SEQ ID No.:12* 
EIVLTQSPGTLSLSPGERATLSCRASQSITGSYLAWYQQKPGQAPRLLICGASSWATGIPDR
FSGSGSGTDFTLTISRLEPEDFAVYYCQQYSSSPITFGQGTRLEIKRTVAAPSVFIFPPSDE
QLKSGTASVVCLLNNFYPREAKVQWKVDNALQSGNSQESVTEQDSKDSTYSLSSTLTLSKAD
YEKHKVYACEVTHQGLSSPVTKSFNRGEC
 The boxed residue in this sequence represents an unpaired cysteine (C49) that may be changed to any other amino acid. Examples of such changes are highlighted in the light chain sequences below.
*3.19.3 heavy chain SEQ ID No:14* 
QVQLVESGGGVVQPGRSLRLSCAASGFTFTNYGMHWGRQAPGKGLEWVAVISHDGNNKYYVD
SVKGRFTISRDNSKNTLYLQMNSLRAEDTAVYYCAREGIDFWSGLNWFDPWGQGTLVTVSS
 The boxed residue in this sequence represents an example of a residue that may be "backmutated" to another residue. One example of such a "backmutation" is represented in the MEDI5 heavy chain sequence.
*MEDI1 light chain SEQ ID No:33* 
EIVLTQSPGTLSLSPGERATLSCRASQSITGSYLAWYQQKPGQAPRLLITGASSWATGIPDR
FSGSGSGTDFTLTISRLEPEDFAVYYCQQYSSSPITFGQGTRLEIKRTVAAPSVFIFPPSDE
QLKSGTASVVCLLNNFYPREAKVQWKVDNALQSGNSQESVTEQDSKDSTYSLSSTLTLSKAD
YEKHKVYACEVTHQGLSSPVTKSFNRGEC
*MEDI2 light chain SEQ ID No:34* 
EIVLTQSPGTLSLSPGERATLSCRASQSITGSYLAWYQQKPGQAPRLLINGASSWATGIPDR
FSGSGSGTDFTLTISRLEPEDFAVYYCQQYSSSPITFGQGTRLEIKRTVAAPSVFIFPPSDE
QLKSGTASVVCLLNNFYPREAKVQWKVDNALQSGNSQESVTEQDSKDSTYSLSSTLTLSKAD
YEKHKVYACEVTHQGLSSPVTKSFNRGEC
*MEDI3 light chain SEQ ID No:35* 
EIVLTQSPGTLSLSPGERATLSCRASQSITGSYLAWYQQKPGQAPRLLIDGASSWATGIPDR
FSGSGSGTDFTLTISRLEPEDFAVYYCQQYSSSPITFGQGTRLEIKRTVAAPSVFIFPPSDE
QLKSGTASVVCLLNNFYPREAKVQWKVDNALQSGNSQESVTEQDSKDSTYSLSSTLTLSKAD
YEKHKVYACEVTHQGLSSPVTKSFNRGEC
*MEDI4 light chain SEQ ID No:36* 
EIVLTQSPGTLSLSPGERATLSCRASQSITGSYLAWYQQKPGQAPRLLIAGASSWATGIPDR
FSGSGSGTDFTLTISRLEPEDFAVYYCQQYSSSPITFGQGTRLEIKRTVAAPSVFIFPPSDE
QLKSGTASVVCLLNNFYPREAKVQWKVDNALQSGNSQESVTEQDSKDSTYSLSSTLTLSKAD
YEKHKVYACEVTHQGLSSPVTKSFNRGEC
*MEDI5 heavy chain SEQ ID No:37* 
QVQLVESGGGVVQPGRSLRLSCAASGFTFTNYGMHWVRQAPGKGLEWVAVISHDGNNKYYVD
SVKGRFTISRDNSKNTLYLQMNSLRAEDTAVYYCAREGIDFWSGLNWFDPWGQGTLVTVSS
*MEDI6 light chain SEQ ID No:38*

EIVLTQSPGTLSLSPGERATLSCRASQSITGSYLAWYQQKPGQAPRLLITGASSWATGIADR
FSGSGSGTDFTLTISRLEPEDFAVYYCQQYSSSPITFGQGTRLEIKRTVAAPSVFIFPPSDE
QLKSGTASVVCLLNNFYPREAKVQWKVDNALQSGNSQESVTEQDSKDSTYSLSSTLTLSKAD
YEKHKVYACEVTHQGLSSPVTKSFNRGEC

### Method of making antibodies useful in the liquid antibody formulations of the invention

The above-described anti Ang-2 antibodies can be prepared by methods well known to those skilled in the art. These methods may include preparation of the antibody using a hybridoma, recombinant antibodies, humanized antibodies, human antibodies, etc.

Antibodies in the form of antibody fragments, low molecular weight antibodies and modified antibodies can also be employed as the antibody in the present invention. Examples of the antibody fragments and low molecular weight antibodies include Fab, F(ab')₂, Fv, and single chain Fv (scFv, sc(Fv)₂ and the like) having one or more specificities, prepared by ligating the Fvs in the H-chain and L-chain through an appropriate linker (Huston, J. S. et al., Proc. Natl. Acad. Sci. U.S.A. (1988) 85, 5879-5883). Specifically, an antibody is treated with papain or pepsin to generate antibody fragments, or a gene encoding these antibody fragments is constructed, and the gene is expressed in appropriate host cells after introducing the gene into an expression vector (see, for example, Co, M. S. et al., T. Immunol. (1994)152, 2968-2976; Better, M. and Horwitz, A. H.; Methods Enzymol. (1989) 178, 476-496; Pluckthun, A. and Skerra, A., Methods Enzymol. (1989) 178, 497-515; Lamoyi, E., Methods Enzymol, (1986) 121, 652-663; Rousseaux, J. et al., Methods Enzymol. (1986)121, 663-669; Bird, R. E. and Walker, B. W., Trends Biotechnol. (1991) 9, 132-137.

Antibodies bound to various molecules such as polyethylene glycol (PEG) can also be used as modified antibodies. The term "antibody" used in the present invention also includes these modified antibodies. These modified antibodies can be obtained by chemically modifying an obtained antibody. Methods for carrying out the modifications are established in the art.

In one embodiment there is provided a hybridoma that produces the light chain and/or the heavy chain of antibody as described hereinabove. In one embodiment, the hybridoma produces the light chain and/or the heavy chain of a fully human monoclonal antibody. In another embodiment, the hybridoma produces the light chain and/or the heavy chain of the fully human monoclonal antibody 3.19.3, 3.3.2 or 5.88.3. Alternatively the hybridoma produces an antibody which binds to the same epitope or epitopes as fully human monoclonal antibody 3.19.3, 3.3.2 or 5.88.3.

### Methods of treatment

Still further embodiments of the invention include methods of effectively treating a patient suffering from an angiogenesis-related disease by administering to the patient an effective amount of a liquid antibody formulation comprising a fully human monoclonal antibody that specifically binds to Angiopoietin-2 (Ang-2). In one embodiment, the method includes inhibiting Angiopoietin-2 (Ang-2) induced angiogenesis in an animal. In another embodiment, the method includes treating cancer in a patient. In general, these methods include selecting an animal in need of treatment and administering to said animal a liquid antibody formulation of the invention comprising a therapeutically effective dose of a fully human monoclonal antibody wherein said antibody specifically binds to Ang-2. The anti-Ang-2 antibody can be administered alone, or can be administered in combination with additional antibodies or chemotherapeutic drug or radiation therapy. The method can be performed *in vivo.* In one embodiment, the patient is a human patient.

In one embodiment, the invention is directed towards use of a liquid antibody formulation described herein in the preparation of a medicament for the treatment angiogenesis-related diseases. In another embodiment, the liquid antibody formulations described herein can be used for the preparation of a medicament for the effective treatment of disease-related angiogenesis in an animal. In a more specific embodiment, the formulation can be used for the preparation of a medicament for the effective treatment of Angiopoietin-2 induced angiogenesis in a patient.

Disease-related angiogenesis may be any abnormal, undesirable or pathological angiogenesis, for example tumor-related angiogenesis. Angiogenesis-related diseases include, but are not limited to, non-solid tumors such as leukaemia, multiple myeloma or lymphoma, and also solid tumors such as melanoma, small cell lung cancer, non-small cell lung cancer, glioma, hepatocellular (liver) carcinoma, glioblastoma, carcinoma of the thyroid, bile duct, bone, gastric, brain/CNS, head and neck, hepatic, stomach, prostate, breast, renal, testicular, ovarian, skin, cervical, lung, muscle, neuronal, oesophageal, bladder, lung, uterine, vulval, endometrial, kidney, colorectal, pancreatic, pleural/peritoneal membranes, salivary gland, gastric (stomach) cancer, esophageal carcinoma, mesothelioma, sarcomas, biliary (cholangiocarcinoma), small bowel adenocarcinoma, pediatric malignancies and epidermoid carcinoma.

### Modes of Delivery

The liquid antibody formulation of the invention can be administered using any suitable route in accord with known methods, e.g., injection or infusion by intravenous, intraperitoneal, intracerebral, intramuscular, intraocular, intraarterial, intrathecal, or intralesional routes. In a more specific embodiment, the liquid antibody formulation of this invention is suitable for parenteral administration such as intravenous, intradermal, intramuscular, intraperitoneal, or subcutaneous injection.

### Article of Manufacture/Kits

The invention also provides a pharmaceutical kit or article of manufacture. In one embodiment, the kit or article of manufacture can include primary and secondary packaging components which can be referred to as a packaging system. The term "packaging component" refers to any single part of the packaging system. Packaging components can include containers (e.g., ampules, vials, bottles), container liners, closures (e.g., screw caps, stoppers), closure liners, stopper overseals, container inner seals, administration ports, overwraps, administration accessories, and container labels. The term "primary packaging component" refers to a packaging component that is or may be in direct contact with the liquid formulation. The term "secondary packaging component" refers to a packaging component that is not and will not be in direct contact with the liquid formulation. Materials of construction refer to the materials used to manufacture a packaging component.

Preferably, primary packaging components are compatible with the liquid formulation and will not cause unacceptable changes in the quality of the active agent or the packaging component. Examples of changes in quality include, for example, loss of potency due to absorption or adsorption of the active agent by a packaging component, or degradation of the active agent induced by a chemical entity leached from a packaging component; reduction in the concentration of an excipient due to absorption, adsorption or leachable-induced degradation; precipitation; changes in drug product pH; discoloration of either the dosage form or the packaging component; or increase in brittleness of the packaging component. Additionally, preferred packaging components are constructed of materials that will not leach harmful or undesirable amounts of substances.

In one embodiment, the kit includes one or more non-glass primary packaging components. In a more particular embodiment, the kit includes one or more non-glass containers, for example, an ampoule, vial or bottle. In general, the term "glass" refers to an amorphous (non-crystalline) solid material. Typically the term "glass" is applied to inorganic solids (as compared to plastics or other organic materials). Most glasses are hard and brittle solids. In a more particular embodiment, the term "glass" refers to an amorphous solid material made of Silicon Dioxide (SiO₂) and, optionally, additives, including but not limited to additives to adjust coefficient of thermal expansion, color, conductivity, and melting point. Examples of typical additives include, but are not limited to boron, phosphorus, lead, sodium carbonate, calcium oxide, magnesium oxide, aluminum oxide or combinations thereof. In one embodiment, the term "glass" refers to an amorphous solid material that includes at least about 60%, 65% 70%, 75%, 80%, 85%, and up to 90% silicon dioxide. One chemical composition for glass includes between about 70% and about 80% silicon dioxide (SiO₂), between about 10% and about 11% boron trioxide (B₂O₃ between about 1% and about 10% aluminum oxide (Al₂O₃), between about 5% and about 10% sodium oxide (Na₂O), and between about 1 % and about 2% calcium oxide (CaO). The term "glass" can also refer to coated glass packaging materials.

The term "non-glass" refers to polymeric materials and can include various natural and synthetic polymers. Polymeric materials not only reduce the risk of the container breaking, but also reduce concerns relating to glass delamination and alkali leachates. Additionally, it is believed that non-glass materials can reduce antibody aggregation. While not wishing to be bound by theory, it is believed that the surface interaction between a hydrophobic monoclonal antibody and the silicon in the glass container or in a silicone coating on the surface of the glass container can interact with hydrophobic protein domains and increase aggregation. Examples of non-glass polymers include, but are not limited to polypropylene (PP), polyethylene terephthalate (PETG), high-density polyethylene (HDPE), polyethylene terephthalate (PET), polypentafluorostyrene (PFS), polycarbonate, polyvinyl chloride (PVC), and synthetic plastic resins such as CZ® (polycyclopentane, Daikyo Seiko) and Topas® COC (cyclic olefin copolymer, Ticona) and combinations and copolymers thereof. In a more specific embodiment, the non-glass container, for example, ampoule, vial or bottle is manufactured using polypropylene (PP), high-density polyethylene (HDPE) or polyethylene terephthalate (PETG).

In one embodiment, the kit includes a container, for example, an ampoule, vial or bottle that reduces the exposure of the liquid formulation to air during storage. In one embodiment, the kit reduces the air to liquid ratio of the liquid formulation within the container. The air to liquid ratio can be quantified by the total volume of liquid in a container and the volume of headspace occupied by air. Reduction in air to liquid ratio can be accomplished by increasing the fill volume of the liquid and thereby decrease the volume of air in the container. In one embodiment, the air to liquid ratio (volumeₐᵢᵣ to volume_{liquid}) is less than or equal to 0.1, less than or equal to 0.09, less than or equal to 0.08, less than or equal to 0.07, less than or equal to 0.06, less than or equal to 0.05, less than or equal to 0.04, less than or equal to 0.03, less than or equal to 0.02, or less than or equal to 0.01. In one embodiment, the air to liquid ratio (volumeₐᵢᵣ to volume_{liquid}) is less than or equal to 0.05.

In another embodiment, the kit includes at least one primary packaging component that is a container closure. In one embodiment, the container closure reduces the exposure of the liquid formulation to air. In one embodiment, the container is a glass vial. In one embodiment, the container closure is a stopper. In one embodiment, the stopper is a rubber stopper. In a more particular embodiment, the container closure is a coated rubber stopper, for example, a rubber stopper with a fluorocarbon film coating. In one embodiment, the stopper is constructed from a base material that includes chlorobutyl rubber compound. In a more particular embodiment, the chlorobutyl rubber compound contains no dry natural rubber. In one embodiment, the stopper can include a barrier film, for example, West's FluroTec^{®} barrier film, Teflon®, or LyoTec™-coatings, which can help reduce drug/closure interaction. In one embodiment, the stopper complies with compendia including, for example, United States Pharmacopeia (USP), European Pharmacopoea (Ph Eur) and Japanese Pharmacopoeia (JP) compendia. In one embodiment, the rubber stopper is 4432/50 available from West Pharmaceutical Services, Inc. (Lionville, PA).

In one embodiment, the container contains a liquid antibody formulation of the invention and a package insert or label. In one embodiment, the invention provides a kit or article of manufacture that includes a liquid antibody formulation of the invention comprising a stable liquid formulation of an antibody (including antibody fragments thereof) formulated for parenteral administration (e.g., intradermally, intramuscularly, intraperitoneally, intravenously and subcutaneously). In a more specific embodiment, the invention provides a kit or article of manufacture that includes a container containing a liquid formulation of an anti-angiopoietin-2 antibody and a package insert or label which indicates that the composition can be used to treat angiogenesis-related diseases characterized by the overexpression of angiopoietin-2. In the interest of brevity, only a subset of the anti-angiopoietin-2 antibodies described above will be enumerated in this section. However, it is to be understood that the kit or article of manufacture can include any antibody, including but not limited to the anti-angiopoietin-2 antibodies discussed in other sections of this application.

In one embodiment, the invention provides a kit or article of manufacture that includes an antibody liquid formulation of the invention. The antibody in the liquid antibody formulation of the invention may be a monoclonal antibody that binds angiopoietin-2 wherein the antibody includes a variable light chain having a sequence selected from: SEQ ID NO: 12, SEQ ID NO: 20, SEQ ID NO: 32, SEQ ID NO: 28, SEQ ID NO: 4, and SEQ ID NO: 16. The antibody may be a monoclonal antibody that binds angiopoietin-2 wherein the antibody includes a variable heavy chain having a sequence selected from: SEQ ID NO: 10, SEQ ID NO: 18, SEQ ID NO: 30, SEQ ID NO: 26, SEQ ID NO: 2, and SEQ ID NO: 14. The antibody may be a monoclonal antibody that binds angiopoietin-2 wherein the antibody includes a variable light chain having a sequence selected from: SEQ ID NO: 12, SEQ ID NO: 20, SEQ ID NO: 32, SEQ ID NO: 28, SEQ ID NO: 4, and SEQ ID NO: 16 and variable heavy chain having a sequence selected from: SEQ ID NO: 10, SEQ ID NO: 18, SEQ ID NO: 30, SEQ ID NO: 26, SEQ ID NO: 2, and SEQ ID NO: 14.

In one embodiment, the invention provides a kit or article of manufacture that includes a liquid antibody formulation which includes a monoclonal antibody that binds angiopoietin-2 wherein the monoclonal antibody is selected from mAb 3.19.3 (ATCC Accession Number PTA-7260); mAb 3.3.2 (ATCC Accession Number PTA-7258); or mAb 5.88.3 (ATCC Accession Number PTA-7259).

In one embodiment, the invention provides a kit or article of manufacture that includes a liquid antibody formulation which includes a monoclonal antibody that binds angiopoietin-2 The monoclonal antibody includes heavy chain CDR1, CDR2, CDR3 having a sequence selected from: SEQ ID NO: 10, SEQ ID NO: 18, SEQ ID NO: 30, SEQ ID NO: 26, SEQ ID NO: 2, and SEQ ID NO: 14; and/or light chain CDR1, CDR2, CDR3 having a sequence selected from: SEQ ID NO: 12, SEQ ID NO: 20, SEQ ID NO: 32, SEQ ID NO: 28, SEQ ID NO: 4, and SEQ ID NO: 16.

As used herein, the term "about" is used to modify, for example, the quantity of an ingredient in a composition, concentration, volume, process temperature, process time, yield, flow rate, pressure, and ranges thereof, employed in describing the invention. The term "about" refers to variation in the numerical quantity that can occur, for example, through typical measuring and handling procedures used for making compounds, compositions, concentrates or use formulations; through inadvertent error in these procedures; through differences in the manufacture, source, or purity of starting materials or ingredients used to carry out the methods, and other similar considerations. The term "about" also encompasses amounts that differ due to aging of a formulation with a particular initial concentration or mixture, and amounts that differ due to mixing or processing a formulation with a particular initial concentration or mixture. Where modified by the term "about" the claims appended hereto include such equivalents.

It should be noted that, as used in this specification and the appended claims, the singular forms "a," "an," and "the" include plural referents unless the content clearly dictates otherwise. It should also be noted that the term "or" is generally employed in its sense including "and/or" unless the content clearly dictates otherwise.

It should also be noted that, as used in this specification and the appended claims, the phrase "configured" describes a system, apparatus, or other structure that is constructed or configured to perform a particular task or adopt a particular configuration. The phrase "configured" can be used interchangeably with other similar phrases such as "arranged", "arranged and configured", "constructed and arranged", "constructed", "manufactured and arranged", and the like.

All publications and patent applications in this specification are indicative of the level of ordinary skill in the art to which this invention pertains. All publications and patent applications are herein incorporated by reference to the same extent as if each individual publication or patent application was specifically and individually indicated by reference.

This application is intended to cover adaptations or variations of the present subject matter. It is to be understood that the above description is intended to be illustrative, and not restrictive. It should be readily apparent that any one or more of the design features described herein may be used in any combination with any particular configuration. With use of a molding process, such design features can be incorporated without substantial additional manufacturing costs. That the number of combinations are too numerous to describe, and the present invention is not limited by or to any particular illustrative combination described herein. The scope of the present subject matter should be determined with reference to the appended claims, along with the full scope of equivalents to which such claims are entitled.

### EMBODIMENTS

1. A liquid antibody formulation having an improved aggregation profile, the liquid formulation comprising:
   a fully human monoclonal antibody that binds to angiopoietin-2; and
   a pharmaceutically acceptable carrier;
   wherein no more than about 5% of the antibody in the formulation forms an aggregate.
2. The liquid antibody formulation of embodiment 1, wherein the antibody is selected from mAb 3.19.3 (ATCC Accession Number PTA-7260); mAb 3.3.2 (ATCC Accession Number PTA-7258); and mAb 5.88.3 (ATCC Accession Number PTA-7259).
3. The liquid antibody formulation of embodiment 1, wherein the antibody is a fully human monoclonal antibody comprising a variable light chain selected from: a light chain sequence comprising SEQ ID NO: 12; a light chain sequence comprising SEQ ID NO: 20; and a light chain sequence comprising SEQ ID NO: 32.
4. The liquid antibody formulation of embodiment 1, wherein the antibody is a fully human monoclonal antibody comprising a variable heavy chain selected from: a heavy chain sequence comprising SEQ ID NO: 10; a heavy chain sequence comprising SEQ ID NO: 18; and a heavy chain sequence comprising SEQ ID NO: 30.
5. The liquid antibody formulation of embodiment 1, wherein the antibody is a fully human monoclonal antibody comprising a variable light chain and a variable heavy chain member selected from:
   a light chain sequence comprising SEQ ID NO: 12 and a heavy chain sequence comprising SEQ ID NO: 10;
   a light chain sequence comprising SEQ ID NO: 20 and a heavy chain sequence comprising SEQ ID NO: 18; and
   a light chain sequence comprising SEQ ID NO: 32 and a heavy chain sequence comprising SEQ ID NO: 30.
6. The liquid antibody formulation of embodiment 1, wherein the antibody is a fully human monoclonal antibody selected from an antibody comprising heavy chain CDR1, CDR2, CDR3 of SEQ ID NO: 10 and light chain CDR1, CDR2, CDR3 of SEQ ID NO: 12; heavy chain CDR1, CDR2, CDR3 of SEQ ID NO: 18 and light chain CDR1, CDR2, CDR3 of SEQ ID NO: 20; and heavy chain CDR1, CDR2, CDR3 of SEQ ID NO: 30 and light chain CDR1, CDR2, CDR3 of SEQ ID NO: 32.
7. The liquid antibody formulation of embodiment 1, wherein the antibody comprises a variable light chain comprising a sequence selected from SEQ ID No:33 (MEDI1); SEQ ID No:34 (MEDI2); SEQ ID No:35 (MEDI3); SEQ ID No:36 (MEDI4); and SEQ ID No:38 (MEDI6).
8. The liquid antibody formulation of embodiment 7, wherein the antibody further comprises a variable heavy chain region comprising SEQ ID No:37 (MEDI5).
9. The liquid antibody formulation of any one of embodiments 1-8, wherein the liquid antibody formulation has a pH between about 5.0 and about 8.0.
10. The liquid antibody formulation of any one of embodiments 1-9, wherein the antibody aggregate comprises visible particles, subvisible particles, precipitates, fibrils, or a combination thereof.
11. The liquid antibody formulation of embodiment 10, wherein the aggregate comprises sub-visible particles greater than at least about 2 µm and up to about 200 µm.
12. The liquid antibody formulation of any one of embodiments 1-11, wherein the pharmaceutically acceptable carrier comprises distilled water.
13. The liquid antibody formulation of any one of embodiments 1-12, comprising at least about 0% and up to about 1% of a surfactant.
14. The liquid antibody formulation of embodiment 13, wherein the surfactant is a nonionic surfactant selected from polysorbate, poloxamer and combinations thereof.
15. The liquid antibody formulation of embodiment 14, wherein polysorbate is selected from polysorbate 20, polysorbate 80, and combinations thereof.
16. The liquid antibody formulation of embodiment 15, wherein the polysorbate is polysorbate 20.
17. An article of manufacture comprising:
   one or more non-glass primary packaging components comprising one or more containers containing a liquid antibody formulation having an improved aggregation profile, the liquid formulation comprising a fully human monoclonal antibody that binds to angiopoietin-2 in a pharmaceutically acceptable carrier;
   wherein no more than about 5% of the antibody in the formulation forms an aggregate.
18. The article of manufacture of embodiment 17, wherein one or more containers are non-glass containers selected from an ampule, vial, bottle and combinations thereof.
19. The article of manufacture of embodiments 17 or 18, wherein the non-glass container comprises polymer selected from polypropylene (PP), polyethylene terephthalate (PETG), high-density polyethylene (HDPE), polyethylene terephthalate (PET), polypentafluorostyrene (PFS), polycarbonate, polyvinyl chloride (PVC), polycyclopentane (CZ®), cyclic olefin copolymer (COC), and combinations or copolymers thereof.
20. The article of manufactured of embodiment 19, wherein the non-glass container comprises polypropylene.
21. The article of manufacture of any one of embodiments 17-20, wherein the container contains air and the liquid formulation in a ratio of volumeₐᵢᵣ to volume_{liquid} ratios of less than or equal to 0.05.
22. The article of manufacture of any one of embodiments 17-21, wherein at least one primary packaging component comprises a container closure selected from polypropylene (PP), polyethylene terephthalate (PETG), high-density polyethylene (HDPE), polyethylene terephthalate (PET), polypentafluorostyrene (PFS), polycarbonate, polyvinyl chloride (PVC), polycyclopentane (CZ®), cyclic olefin copolymer (COC), and combinations or copolymers thereof.
23. The article of manufacture of any one of embodiments 17-22, wherein the fully human monoclonal antibody that binds to angiopoietin-2 is selected from mAb 3.19.3 (ATCC Accession Number PTA-7260); mAb 3.3.2 (ATCC Accession Number PTA-7258); and mAb 5.88.3 (ATCC Accession Number PTA-7259).
24. The article of manufacture of any one of embodiments 17-23, wherein the fully human monoclonal antibody comprises a variable light chain selected from: a light chain sequence comprising SEQ ID NO: 12; a light chain sequence comprising SEQ ID NO: 20; and a light chain sequence comprising SEQ ID NO: 32.
25. The article of manufacture of any one of embodiments 17-23, wherein the fully human monoclonal antibody comprises a variable heavy chain selected from: a heavy chain sequence comprising SEQ ID NO: 10; a heavy chain sequence comprising SEQ ID NO: 18; and a heavy chain sequence comprising SEQ ID NO: 30.
26. The article of manufacture of any one of embodiments 17-23, wherein the fully human monoclonal antibody comprises a variable light chain and a variable heavy chain member selected from:
   a light chain sequence comprising SEQ ID NO: 12 and a heavy chain sequence comprising SEQ ID NO: 10;
   a light chain sequence comprising SEQ ID NO: 20 and a heavy chain sequence comprising SEQ ID NO: 18; and
   a light chain sequence comprising SEQ ID NO: 32 and a heavy chain sequence comprising SEQ ID NO: 30.
27. The article of manufacture of any one of embodiments 17-23, wherein the fully human monoclonal antibody is selected from an antibody comprising heavy chain CDR1, CDR2, CDR3 of SEQ ID NO: 10 and light chain CDR1, CDR2, CDR3 of SEQ ID NO: 12; heavy chain CDR1, CDR2, CDR3 of SEQ ID NO: 18 and light chain CDR1, CDR2, CDR3 of SEQ ID NO: 20; and heavy chain CDR1, CDR2, CDR3 of SEQ ID NO: 30 and light chain CDR1, CDR2, CDR3 of SEQ ID NO: 32.
28. The article of manufacture of any one of embodiments 17-23, wherein the fully human monoclonal antibody comprises a variable light chain comprising a sequence selected from SEQ ID No:33 (MEDI1); SEQ ID No:34 (MEDI2); SEQ ID No:35 (MEDI3); SEQ ID No:36 (MEDI4); and SEQ ID No:38 (MEDI6).
29. The article of manufacture of embodiment 28, wherein the fully human monoclonal antibody further comprises a variable heavy chain region comprising SEQ ID No:37 (MEDI5).
30. The article of manufacture of any one of embodiments 17-29, wherein the liquid formulation has a pH between about 5.0 and about 8.0.
31. The article of manufacture of any one of embodiments 17-30, wherein the antibody aggregate comprises visible particles, subvisible particles, precipitates, fibrils, or a combination thereof.
32. The article of manufacture of embodiment 31, wherein the aggregate comprises sub-visible particles greater than at least about 2 µm and up to about 200 µm.
33. The article of manufacture of any one of embodiments 17-32, wherein the pharmaceutically acceptable carrier comprises distilled water.
34. The article of manufacture of any one of embodiments 17-33, comprising at least about 0% and up to about 1 % of a surfactant.
35. The article of manufacture of embodiment 34, wherein the surfactant is a nonionic surfactant selected from polysorbate, poloxamer and combinations thereof.
36. The article of manufacture of embodiment 35, wherein polysorbate is selected from polysorbate 20 (PS-20), polysorbate 80, and combinations thereof.
37. The article of manufacture of embodiment 36, wherein the polysorbate is PS-20.
38. The article of manufacture of embodiment 17, wherein the liquid antibody formulation comprises at least one monoclonal antibody in between about 0.01% and 0.05% polysorbate-20, at a pH of between about 5.0 and 8.0.
39. A method of improving the aggregation profile of a liquid antibody formulation, the method comprising:
   dispensing a quantity of liquid antibody formulation into one or more non-glass containers, the liquid formulation comprising a fully human monoclonal antibody that binds to angiopoietin-2 in a pharmaceutically acceptable carrier;
   storing the liquid antibody formulation in the container for a desired period of time, wherein no more than about 5% of the antibody in the formulation forms an aggregate during the storage time period.
40. The method of any one of embodiment 39, wherein the non-glass container comprises polymer selected from polypropylene (PP), polyethylene terephthalate (PETG), high-density polyethylene (HDPE), polyethylene terephthalate (PET), polypentafluorostyrene (PFS), polycarbonate, polyvinyl chloride (PVC), polycyclopentane (CZ®), cyclic olefin copolymer (COC), and combinations or copolymers thereof.
41. The method of embodiments 39 or 40, wherein the quantity of liquid antibody formulation is dispensed into the container to provide an air to liquid ratio of volumeₐᵢᵣ to volume_{liquid} of less than or equal to 0.05.
42. The method of any one of embodiments 39 - 41, further comprising closing the container with a container closure capable of reducing exposure of the liquid formulation to air polymeric container types including polypropylene (PP), polyethylene terephthalate (PETG), high-density polyethylene (HDPE), polyethylene terephthalate (PET), polypentafluorostyrene (PFS), polycarbonate, polyvinyl chloride (PVC), crystal zenith (CZ®) cyclic olefin copolymer (COC), and combinations or copolymers thereof.
43. The method of any one of embodiments 39 - 42, wherein the antibody is selected from mAb 3.19.3 (ATCC Accession Number PTA-7260); mAb 3.3.2 (ATCC Accession Number PTA-7258); and mAb 5.88.3 (ATCC Accession Number PTA-7259).
44. The method of any one of embodiments 39 - 42, wherein the antibody is a fully human monoclonal antibody comprising a variable light chain selected from: a light chain sequence comprising SEQ ID NO: 12; a light chain sequence comprising SEQ ID NO: 20; and a light chain sequence comprising SEQ ID NO: 32.
45. The method of any one of embodiments 39 - 42, wherein the antibody is a fully human monoclonal antibody comprising a variable heavy chain selected from: a heavy chain sequence comprising SEQ ID NO: 10; a heavy chain sequence comprising SEQ ID NO: 18; and a heavy chain sequence comprising SEQ ID NO: 30.
46. The method of any one of embodiments 39 - 42, wherein the antibody is a fully human monoclonal antibody comprising a variable light chain and a variable heavy chain member selected from:
   a light chain sequence comprising SEQ ID NO: 12 and a heavy chain sequence comprising SEQ ID NO: 10;
   a light chain sequence comprising SEQ ID NO: 20 and a heavy chain sequence comprising SEQ ID NO: 18; and
   a light chain sequence comprising SEQ ID NO: 32 and a heavy chain sequence comprising SEQ ID NO: 30.
47. The method of any one of embodiments 39 - 42, wherein the antibody is a fully human monoclonal antibody selected from an antibody comprising heavy chain CDR1, CDR2, CDR3 of SEQ ID NO: 10 and light chain CDR1, CDR2, CDR3 of SEQ ID NO: 12; heavy chain CDR1, CDR2, CDR3 of SEQ ID NO: 18 and light chain CDR1, CDR2, CDR3 of SEQ ID NO: 20; and heavy chain CDR1, CDR2, CDR3 of SEQ ID NO: 30 and light chain CDR1, CDR2, CDR3 of SEQ ID NO: 32.
48. The method of embodiment 47, wherein the antibody comprises a variable light chain comprising a sequence selected from SEQ ID No:33 (MEDI1); SEQ ID No:34 (MEDI2); SEQ ID No:35 (MEDI3); SEQ ID No:36 (MEDI4); and SEQ ID No:38 (MEDI6).
49. The method of embodiment 48, wherein the antibody further comprises a variable heavy chain region comprising SEQ ID No:37 (MEDI5).
50. The method of any one of embodiments 39 - 49, wherein the liquid formulation comprises a non-zwitterionic buffer selected from phosphate, tris, citrate, succinate, acetate, or a combination thereof.
51. The method of any one of embodiments 39 - 50, wherein the liquid antibody formulation has a pH between about 5.0 and about 8.0.
52. The method of any one of embodiments 39 - 51, wherein the antibody aggregate comprises visible particles, subvisible particles, precipitates, fibrils, or a combination thereof.
53. The method of embodiment 52, wherein the aggregate comprises sub-visible particles greater than at least about 2 µm and up to about 200 µm.
54. The method of any one of embodiments 39 - 53, wherein the pharmaceutically acceptable carrier comprises distilled water.
55. The method of any one of embodiments 39 - 54, comprising at least about 0% and up to about 1% of a surfactant.
56. The method of embodiment 55, wherein the surfactant is a nonionic surfactant selected from polysorbate, poloxamer and combinations thereof.
57. The method of embodiment 56, wherein polysorbate is selected from polysorbate 20 (PS-20), polysorbate 80, and combinations thereof.
58. The method of embodiment 57, wherein the polysorbate is polysorbate 20.
59. The method of embodiment 58, wherein the liquid formulation comprises between about 0 % and 0.05% polysorbate-20, at a pH of between about 5.0 and 8.0.
60. A method of improving the aggregation profile of a liquid antibody formulation, the method comprising:
   dispensing a quantity of liquid antibody formulation into one or more non-glass containers, the liquid formulation comprising:
      a fully human monoclonal antibody that binds to angiopoietin-2 in a pharmaceutically acceptable carrier;
      storing the liquid antibody formulation in the container for a desired period of time, wherein no more than about 5% of the antibody in the formulation forms an aggregate during the storage time period.

### EXAMPLES

The following examples are offered to illustrate this invention and not to be construed in any way as limiting the scope of the invention.

The interface between a liquid antibody formulation and various surfaces, including glass, polystyrene, polypropylene, polyethylene, polyethylene terephthalate, polytetrafluoroethylene and cyclic olefin polymer, was investigated to determine the effect of the surfaces on visible particle formation. Sub-visible particle (SVP) analysis of a liquid antibody formulation was performed using FlowCAM (Fluid Imagining Technologies; Serial# 526). Samples were analyzed using a 4X objective and FlowCell with an upper limit of 300 µm. Visible particle (VP) counts were monitored over time by visual analysis. All glass vials used were Type 1 Borosilicate 5CC vials (West) and all stoppers used were 20 mm Tef2 serum stoppers (West-4432/50GY). All studies were performed in triplicates and had buffer controls for each of the conditions, variables and temperatures evaluated.

Unless indicated otherwise, various liquid formulation of IgG1 monoclonal antibody (MEDI1/5 - IgG1) were examined. The monoclonal antibody was produced by recombinant DNA technology.

### Example 1: Investigating Interfaces: Liquid/Air, Liquid/Glass and Liquid/Rubber

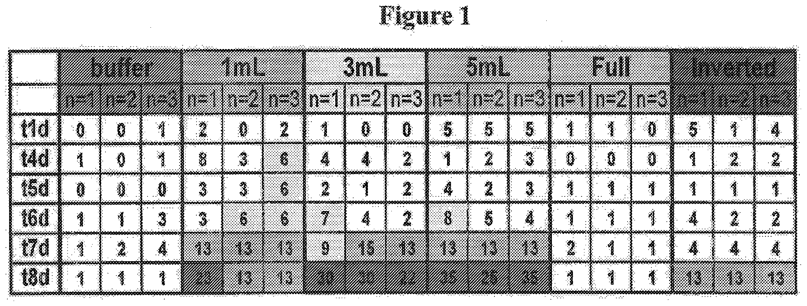

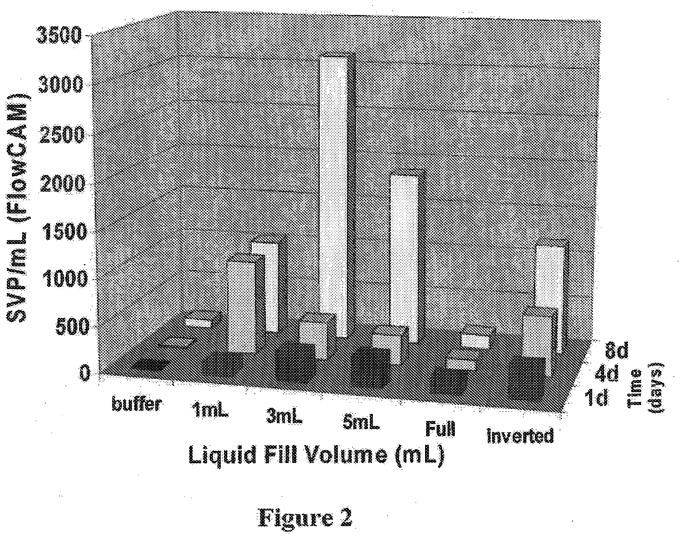

The purpose of this investigation was to evaluate the effect of varying the air to liquid ratio in a storage container. Fill volumes of 1 mL, 3 mL, 5 mL, and full (all upright), and 3 mL (inverted) in a 5 cc vial were evaluated in triplicate with an antibody concentration of 1 mg/mL at 5°C over 8 days. Full vials, with the least air exposure, had the lowest visible particle (VP) (Figure 1) and sub-visible particle (SVP) counts (Figure 2). Visible particles were identified using visual inspection. The vials were held against the black background of a black and white light box (Apollo II Light Viewer, Adelphi Manufacturing Co Ltd., Cat.# 6031200), with an illumination of ∼ 3400 Lux, and the total number of particles in the vials were approximated. For assays with varying fill volumes, all visible particle counts were normalized to reflect visible particles per mL of sample. Sub visible particles were monitored using FlowCAM (Fluid Imaging Technologies Inc, Serial# 157760), a fluid imaging technique. 1 mL of sample, at concentration, was analyzed using a 4X camera lens and an imaging rate of 5 frames per second. All visible and sub visible assays were performed in duplicates or triplicates. Partially filled vials, with higher air exposure, had higher VP and SVP counts. Samples in inverted vials, in which the formulation was exposed to the rubber stopper, had lower VP and SVP counts as compared to their upright counterparts.

The results of this investigation (shown in Figure 1 and Figure 2) suggest that reducing the exposure of the liquid formulation to air can significantly reduce both visible and sub-visible particle formation. Since contact with the rubber stopper appears to reduce particle formation (as compared to liquid exposed to glass and air only), the effects of polymers on particle formation was then investigated (see, Example 3, below)

### Example 2: Investigating oxidation at the air/liquid interface

The purpose of this investigation was to evaluate the effects of oxidation at the air/liquid interfaces. Briefly, 5CC type 1 borosilicate glass vials (West) were filled with 3 mL of 1 mg/mL of monoclonal antibody MEDI1/5. The control vials were purged with air by bubbling air at a very slow rate into the solution. Sample vials were purged with nitrogen in a similar manner using a similar purging flow rate. VP and SVP particle data was collected for all samples as described above.

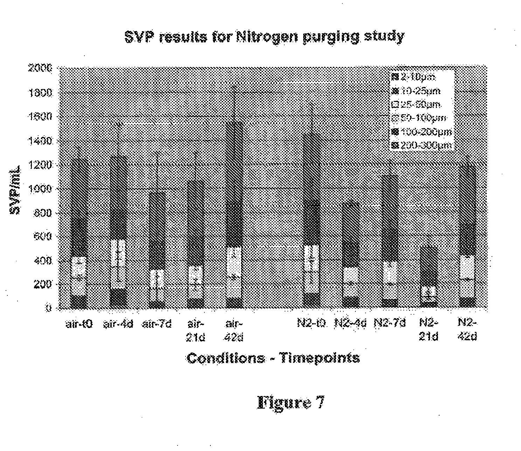

As shown in Figure 7, no significant differences were observed in VP or SVP counts between samples purged with air versus samples purged with nitrogen. As such, it does not appear that oxidation at the air water interface contributes to particle formation.

### Example 3. Investigating interfaces: Liquid/polymer

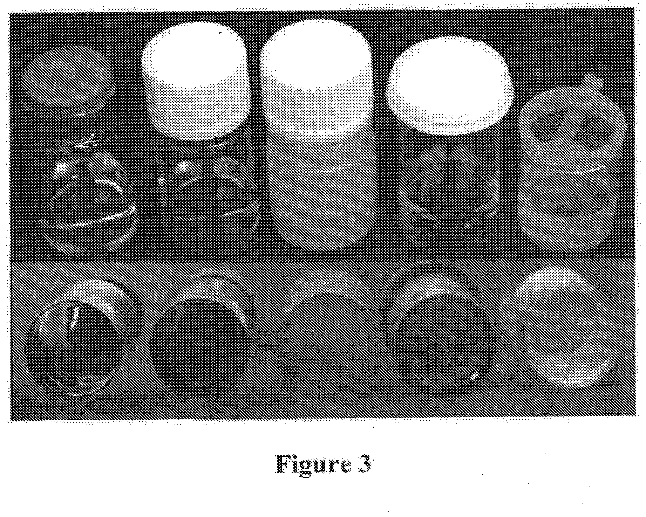

In this investigation, the effect of different container types on particle formation was assessed. The following containers were used: type 1 Borosilicate 5cc glass vials (West), polyethylene terephthalate (PETG) 10 mL bottles, (available from NALGENE (2035 Diagnostic Bottles, Sterile Catalog No. 342035), high-density polyethylene (HDPE) 8 mL narrow mouth vials, sterile, available from NALGENE (Catalog No. 342002-9025), Polystyrene (PS) 3.5 dram vials (available from LA Container, part No. 55-03) and Polypropylene (PP) 1.5 dram microvials (available from LA Container as part No. 071100). The containers are shown in Figure 3.

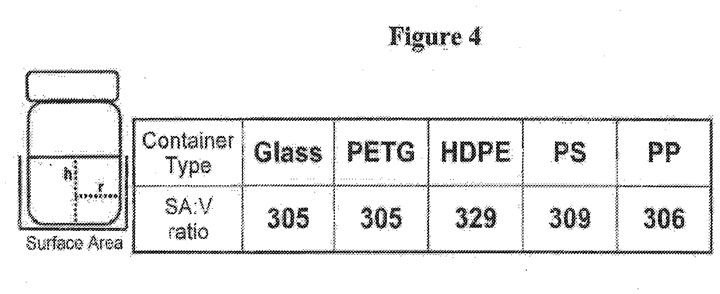

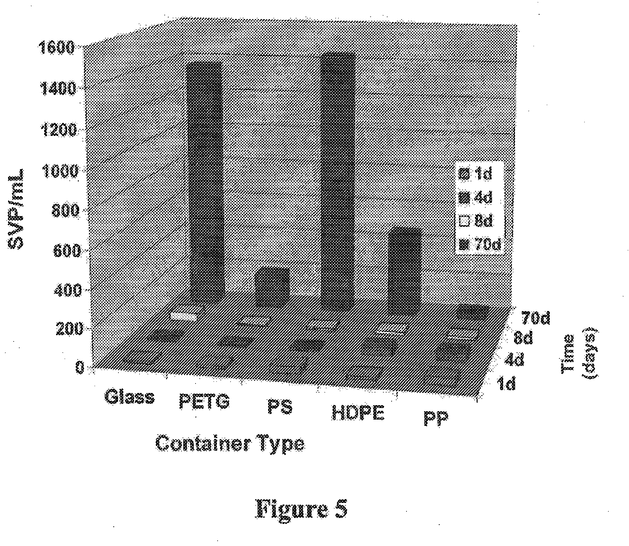

Briefly, the containers were filled with the 1 mg/mL antibody formulation (described above) to similar surface area (SA) to volume (V) ratios (SA:V ratio between 305-329) across all container types (See, Table 4 and Figure 4). The containers were incubated at 5°C and SVP was monitored over a 70 day period. Sub visible particles were monitored using FlowCAM (Fluid Imaging Technologies Inc, Serial# 157760), a fluid imaging technique. 1 mL of sample, at concentration, was analyzed using a 4X camera lens and an imaging rate of 5 frames per second. All visible and sub visible assays were performed in duplicates or triplicates. Samples were swirled in a controlled manner that involved swirling the glass vial gently approximating 4 rotations in 4 seconds followed by one inversion and 2 additional rotations in 2 seconds at regular intervals (approximately every 7 to 14 days) from day 22-day 70 in an attempt to exacerbate particle formation. Glass and PS exposed samples had the highest SVP counts followed by HDPE and PETG. PP showed the least amount of SVP over time (Figure 5).

The data indicates that exposure of the liquid formulation to glass increases SVP counts while contact with other polymers can reduce SVP counts.

**Table 4:**

| **Container Closure** | **measured** | | **Calculated height (mm)** | **Calculated Volume (mm3)** | **Calculated Volume (mL)** | **Calculated SA(mm²)** |
|---|---|---|---|---|---|---|
| | **dia(mm)** | **rad (mm)** | | | | |
| Glass | 20 | 10 | 9.55 | 3000.00 | 3.00 | 914.16 |
| PETG | 20 | 10 | 9.55 | 3000.00 | 3.00 | 914.16 |
| HDPE | 22 | 11 | 6.77 | 2573.50 | 2.57 | 848.04 |
| PS | 20.6 | 10.3 | 8.68 | 2892.97 | 2.89 | 895.03 |
| PP | 16.7 | 8.35 | 15.06 | 3298.74 | 3.30 | 1009.16 |

### Example 4: Introduction of glass and polymer beads into different container types

In this investigation, we examined whether (i) the introduction of glass increases SVP formation and/or (ii) the introduction of polymer reduces SVP formation.

Briefly, different container types were filled with 3 mL of the 1 mg/mL antibody formulation (described above) and beads composed of different materials (glass and various polymers) were introduced into different container types and particle formation was monitored over a 21 day period.

The following beads were used: glass beads (3/8" Borosilicate glass balls; available from Craig Ball Sales, Product number: VEBO.009525.000); polypropylene (PP) balls (available from Engineering Laboratories, Inc., product number BL03750PPNA1CD); polystyrene (PS) balls (available from Engineering Laboratories, Inc., product number BL03125STWT1DC); Teflon™ balls (available from Engineering Laboratories, Inc. product number BL03750TENA2BC); and 13 mm polybutadiene (bromobutyl) rubber stopper. The following containers were evaluated: glass vial (available from West Pharmaceutical Services, product number 6800318) and high density polyethylene (HDPE) narrow mouth bottles (available from Nalgene, product numbers 342002-9025 and 342089) and Crystal Zenith (CZ) cyclic olefin polymer vials.

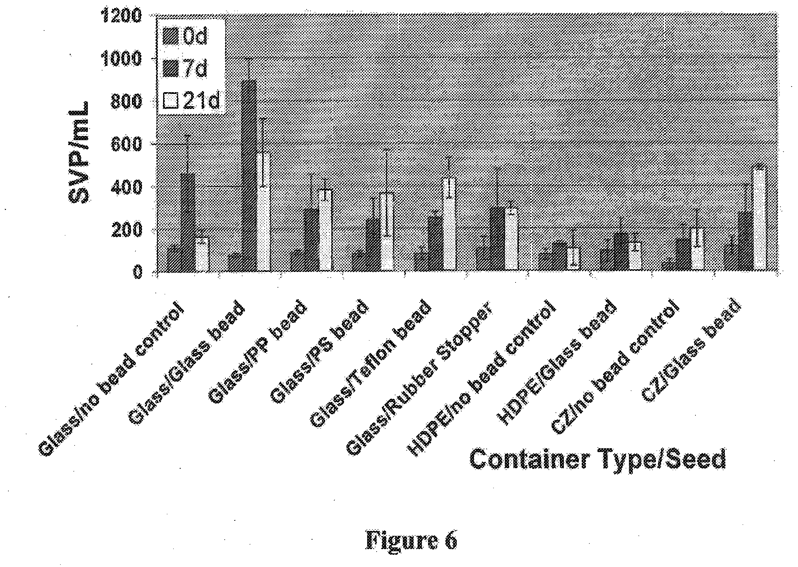

The results (Figure 6) indicate that the addition of polymer beads into the glass containers does not result in significant changes in SVP counts as compared to the glass/no-bead control. This suggests that the polymers do not reduce particle formation. However, polymer/no bead control containers had lower SVP counts than glass/no bead control containers. Additionally, Container/Glass bead conditions had higher SVP counts than control conditions for all three container types with the glass/glass bead condition having the highest SVP counts. This data indicates that exposure to glass may result in increased particle formation.

### Example 5: Surface Tension (ST) measurements:

The surface activity of monoclonal antibody MEDI1/5 was compared to other IgG1 antibodies that are not as prone to forming particles at the air-water interface. Briefly, Surface tension measurements were performed on a tensiometer (Kruss Model K100C; Serial# 20012809) using a 1.2 mL plate and PLO3 rod system. 0.2 mL of sample was placed at the centre of a 1.2 mL plate and measurements were conducted, at room temperature, using a platinum rod (PL03). The detection sensitivity was set to 0.05 and measurements were performed for 300 seconds. System suitability was performed using deionized water (DIW) before and after each experiment.

The results indicate that monoclonal antibody MEDI1/5 may have increased surface activity (ST≈55 mN/m) over a non-particle forming IgG1 mAb.

### Example 6: Surfactant Optimization

Surfactants are known to preferentially bind to interfaces and surfactant containing solutions have high surface activity (0.03% PS20 ST≈39 mN/m). In this study, the effect of surfactant on a monoclonal antibody liquid formulation was examined.

Briefly, 1 mL of MEDI1/5 buffer was filled into each of six 2 cc type 1 borosilicate glass vials. Polysorbate-20 (PS-20) was spiked into each of four out of the 6 vials to achieve final concentrations of: 0.005%, 0.01%, 0.03% and 0.06%. No PS-20 was spiked into the remaining two vials. One of the no-PS20 spiked vials was designated as a control sample and stored at 2-8°C. The other 5 vials were agitated by vortexing at a setting of 6 on the vortex for 4 hours at room temperature. All samples, including the control sample, were then assayed for visible and sub visible particle counts.

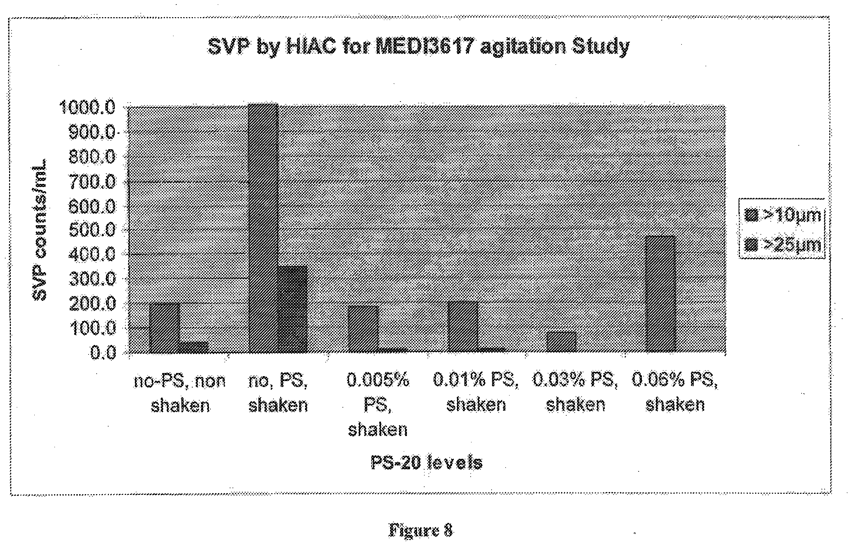

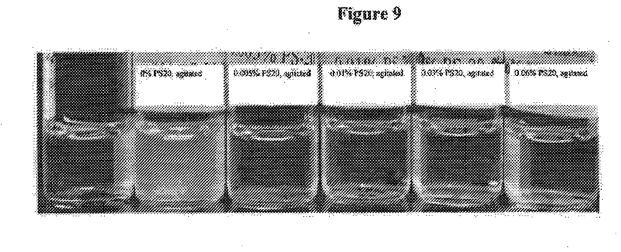

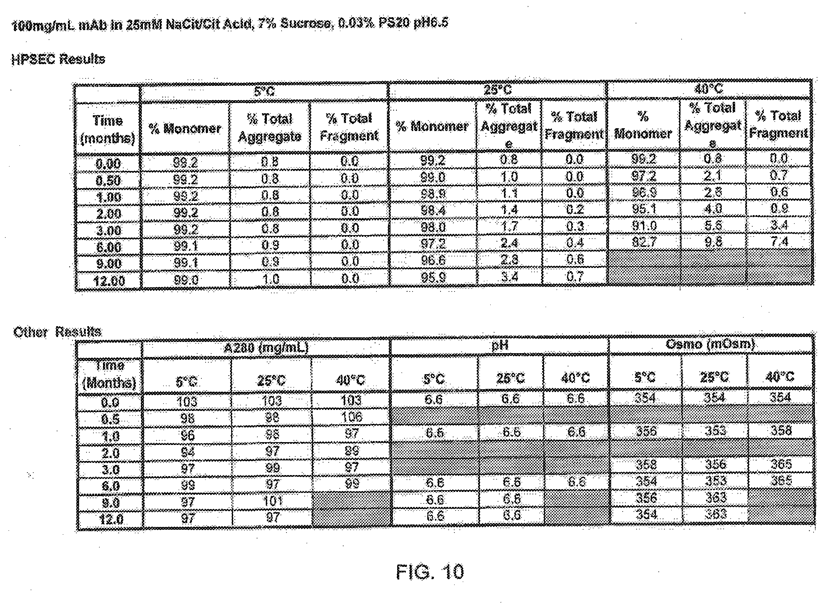

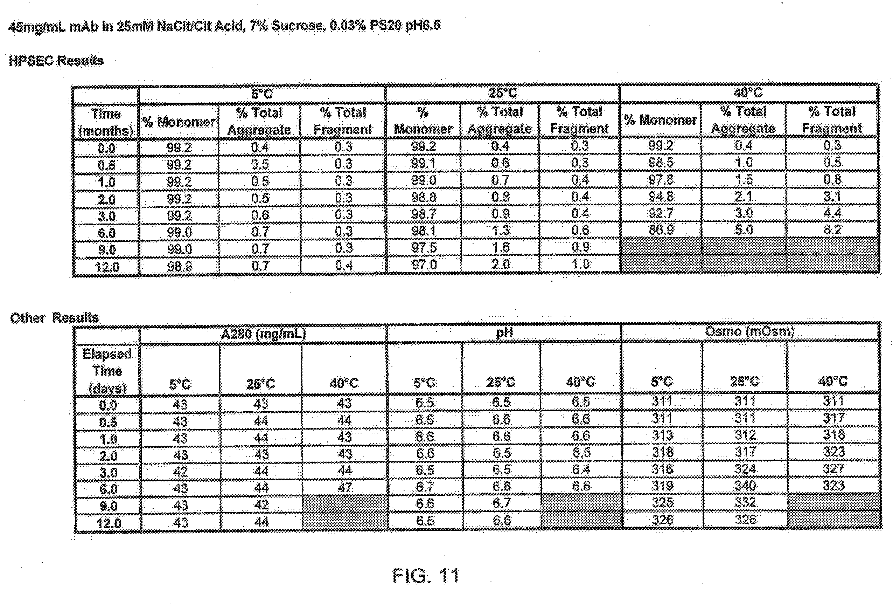

The results indicate that agitation resulted in very high levels of visible and subvisible particle formation in the agitated sample with no PS-20. Addition of any amount of PS-20 was able to reduce the formation of both visible and sub visible particles in MEDI1/5. See, Figures 8 and 9.

SEQUENCE LISTING

MEDIMMUNE, LLC

LIQUID ANTIBODY FORMULATION WITH IMPROVED AGGREGATION PROPERTIES

ANGST-300WO1
PCT/US13/76062
2013-12-18

PCT/US2013/076062
2013-12-18

61/740,193
2012-12-20

38

PatentIn version 3.5

1
366
DNA
Homo sapiens

1
gaggtgcagc tggtggagtc tgggggaggc ttggtccagc ctggggggtc cctgagactc 60

tcctgtgcag tctctggatt cacctttagt agctattgga tgagctgggt ccgccaggct 120

ccagggaagg ggctggagtg ggtggccaac ataaagcaag atggaagtga gaaatactat 180

gtggactctg tgaagggccg attcaccatc tccagagaca acgccaagaa ctcactgtat 240

ctgcaaatga acagcctgag agccgaggac acggctgtgt attactgtgc gagagatcaa 300

ggtatagcag tggctgggcc ctttgactac tggggccagg gaaccctggt caccgtctcc 360

tcagcc 366

2
122
PRT
Homo sapiens

2
Glu Val Gln Leu Val Glu Ser Gly Gly Gly Leu Val Gln Pro Gly Gly
1               5                   10                  15

Ser Leu Arg Leu Ser Cys Ala Val Ser Gly Phe Thr Phe Ser Ser Tyr
            20                  25                  30

Trp Met Ser Trp Val Arg Gln Ala Pro Gly Lys Gly Leu Glu Trp Val
        35                  40                  45

Ala Asn Ile Lys Gln Asp Gly Ser Glu Lys Tyr Tyr Val Asp Ser Val
    50                  55                  60

Lys Gly Arg Phe Thr Ile Ser Arg Asp Asn Ala Lys Asn Ser Leu Tyr
65                  70                  75                  80

Leu Gln Met Asn Ser Leu Arg Ala Glu Asp Thr Ala Val Tyr Tyr Cys
                85                  90                  95

Ala Arg Asp Gln Gly Ile Ala Val Ala Gly Pro Phe Asp Tyr Trp Gly
            100                 105                 110

Gln Gly Thr Leu Val Thr Val Ser Ser Ala
        115                 120

3
322
DNA
Homo sapiens

3
gaaatagtga tgacgcagtc tccagccacc ctgtctgtgt ctccagggga aagagccacc      60

ctctcctgca gggccagtca gactgttagc agcgacttag cctggtacca gcagaaacct     120

ggccaggctc ccaggctcct catctatgga gcatccatta gggccactgg tatcccagcc     180

aggttcagtg gcagtgggtc tgggacagag ttcactctca ccatcagcag cctgcagtct     240

gaagattttg cagtttattc ctgtcagcag tattataact ggtggacgtt cggccaaggg     300

accaaggtgg aaatcaaacg aa                                              322

4
107
PRT
Homo sapiens

4
Glu Ile Val Met Thr Gln Ser Pro Ala Thr Leu Ser Val Ser Pro Gly
1               5                   10                  15

Glu Arg Ala Thr Leu Ser Cys Arg Ala Ser Gln Thr Val Ser Ser Asp
            20                  25                  30

Leu Ala Trp Tyr Gln Gln Lys Pro Gly Gln Ala Pro Arg Leu Leu Ile
        35                  40                  45

Tyr Gly Ala Ser Ile Arg Ala Thr Gly Ile Pro Ala Arg Phe Ser Gly
    50                  55                  60

Ser Gly Ser Gly Thr Glu Phe Thr Leu Thr Ile Ser Ser Leu Gln Ser
65                  70                  75                  80

Glu Asp Phe Ala Val Tyr Ser Cys Gln Gln Tyr Tyr Asn Trp Trp Thr
                85                  90                  95

Phe Gly Gln Gly Thr Lys Val Glu Ile Lys Arg
            100                 105

5
366
DNA
Homo sapiens

5
gaggtgcagc tggtggagtc tgggggaggc ttggtccagc ctggggggtc cctgagactc 60

tcctgtgcag tctctggatt cacctttagt agctattgga tgagctgggt ccgccaggct 120

ccagggaagg ggctggagtg ggtggccaac ataaagcaag atggaagtga gaaatactat 180

gtggactctg tgaagggccg attcaccatc tccagagaca acgccaagaa ctcactgtat 240

ctgcaaatga acagcctgag agccgaggac acggctgtgt attactgtgc gagagatcaa 300

ggtatagcag tggctgggcc ctttgactac tggggccagg gaaccctggt caccgtctcc 360

tcagcc 366

6
122
PRT
Homo sapiens

6
Glu Val Gln Leu Val Glu Ser Gly Gly Gly Leu Val Gln Pro Gly Gly
1               5                   10                  15

Ser Leu Arg Leu Ser Cys Ala Val Ser Gly Phe Thr Phe Ser Ser Tyr
            20                  25                  30

Trp Met Ser Trp Val Arg Gln Ala Pro Gly Lys Gly Leu Glu Trp Val
        35                  40                  45

Ala Asn Ile Lys Gln Asp Gly Ser Glu Lys Tyr Tyr Val Asp Ser Val
    50                  55                  60

Lys Gly Arg Phe Thr Ile Ser Arg Asp Asn Ala Lys Asn Ser Leu Tyr
65                  70                  75                  80

Leu Gln Met Asn Ser Leu Arg Ala Glu Asp Thr Ala Val Tyr Tyr Cys
                85                  90                  95

Ala Arg Asp Gln Gly Ile Ala Val Ala Gly Pro Phe Asp Tyr Trp Gly
            100                 105                 110

Gln Gly Thr Leu Val Thr Val Ser Ser Ala
        115                 120

7
322
DNA
Homo sapiens

7
gaaatagtga tgacgcagtc tccagccacc ctgtctgtgt ctccagggga aagagccacc 60

ctctcctgca gggccagtca gactgttagc agcgacttag cctggtacca gcagaaacct 120

ggccaggctc ccaggctcct catctatgga gcatccatta gggccactgg tatcccagcc 180

aggttcagtg gcagtgggtc tgggacagag ttcactctca ccatcagcag cctgcagtct 240

gaagattttg cagtttattc ctgtcagcag tattataact ggtggacgtt cggccaaggg 300

accaaggtgg aaatcaaacg aa 322

8
107
PRT
Homo sapiens

8
Glu Ile Val Met Thr Gln Ser Pro Ala Thr Leu Ser Val Ser Pro Gly
1               5                   10                  15

Glu Arg Ala Thr Leu Ser Cys Arg Ala Ser Gln Thr Val Ser Ser Asp
            20                  25                  30

Leu Ala Trp Tyr Gln Gln Lys Pro Gly Gln Ala Pro Arg Leu Leu Ile
        35                  40                  45

Tyr Gly Ala Ser Ile Arg Ala Thr Gly Ile Pro Ala Arg Phe Ser Gly
    50                  55                  60

Ser Gly Ser Gly Thr Glu Phe Thr Leu Thr Ile Ser Ser Leu Gln Ser
65                  70                  75                  80

Glu Asp Phe Ala Val Tyr Ser Cys Gln Gln Tyr Tyr Asn Trp Trp Thr
                85                  90                  95

Phe Gly Gln Gly Thr Lys Val Glu Ile Lys Arg
            100                 105

9
366
DNA
Homo sapiens

9
gaggtgcagc tggtggagtc tgggggaggc ttggtccagc ctggggggtc cctgagactc 60

tcctgtgcag tctctggatt cacctttagt agctattgga tgagctgggt ccgccaggct 120

ccagggaagg ggctggagtg ggtggccaac ataaagcaag atggaagtga gaaatactat 180

gtggactctg tgaagggccg attcaccatc tccagagaca acgccaagaa ctcactgtat 240

ctgcaaatga acagcctgag agccgaggac acggctgtgt attactgtgc gagagatcaa 300

ggtatagcag tggctgggcc ctttgactac tggggccagg gaaccctggt caccgtctcc 360

tcagcc 366

10
122
PRT
Homo sapiens

10
Glu Val Gln Leu Val Glu Ser Gly Gly Gly Leu Val Gln Pro Gly Gly
1               5                   10                  15

Ser Leu Arg Leu Ser Cys Ala Val Ser Gly Phe Thr Phe Ser Ser Tyr
            20                  25                  30

Trp Met Ser Trp Val Arg Gln Ala Pro Gly Lys Gly Leu Glu Trp Val
        35                  40                  45

Ala Asn Ile Lys Gln Asp Gly Ser Glu Lys Tyr Tyr Val Asp Ser Val
    50                  55                  60

Lys Gly Arg Phe Thr Ile Ser Arg Asp Asn Ala Lys Asn Ser Leu Tyr
65                  70                  75                  80

Leu Gln Met Asn Ser Leu Arg Ala Glu Asp Thr Ala Val Tyr Tyr Cys
                85                  90                  95

Ala Arg Asp Gln Gly Ile Ala Val Ala Gly Pro Phe Asp Tyr Trp Gly
            100                 105                 110

Gln Gly Thr Leu Val Thr Val Ser Ser Ala
        115                 120

11
322
DNA
Homo sapiens

11
gaaatagtga tgacgcagtc tccagccacc ctgtctgtgt ctccagggga aagagccacc 60

ctctcctgca gggccagtca gactgttagc agcgacttag cctggtacca gcagaaacct 120

ggccaggctc ccaggctcct catctatgga gcatccatta gggccactgg tatcccagcc 180

aggttcagtg gcagtgggtc tgggacagag ttcactctca ccatcagcag cctgcagtct 240

gaagattttg cagtttattc ctgtcagcag tattataact ggtggacgtt cggccaaggg 300

accaaggtgg aaatcaaacg aa 322

12
107
PRT
Homo sapiens

12
Glu Ile Val Met Thr Gln Ser Pro Ala Thr Leu Ser Val Ser Pro Gly
1               5                   10                  15

Glu Arg Ala Thr Leu Ser Cys Arg Ala Ser Gln Thr Val Ser Ser Asp
            20                  25                  30

Leu Ala Trp Tyr Gln Gln Lys Pro Gly Gln Ala Pro Arg Leu Leu Ile
        35                  40                  45

Tyr Gly Ala Ser Ile Arg Ala Thr Gly Ile Pro Ala Arg Phe Ser Gly
    50                  55                  60

Ser Gly Ser Gly Thr Glu Phe Thr Leu Thr Ile Ser Ser Leu Gln Ser
65                  70                  75                  80

Glu Asp Phe Ala Val Tyr Ser Cys Gln Gln Tyr Tyr Asn Trp Trp Thr
                85                  90                  95

Phe Gly Gln Gly Thr Lys Val Glu Ile Lys Arg
            100                 105

13
372
DNA
Homo sapiens

13
caggtgcagc tggtggagtc tgggggaggc gtggtccagc ctgggaggtc cctgagactc      60

tcctgtgcag cctctggatt caccttcact aactatggca tgcactgggg ccgccaggct     120

ccaggcaagg ggctggagtg ggtggcagtt atatcacatg atggaaataa taagtattat     180

gtagactccg tgaagggccg attcaccatc tccagagaca attccaagaa cacgctgtat     240

ctgcaaatga acagcctgag agctgaggac acggctgtgt attactgtgc gagagaggga     300

atcgattttt ggagtggcct caactggttc gacccctggg gccagggaac cctggtcacc     360

gtctcctcag cc                                                         372

14
124
PRT
Homo sapiens

14
Gln Val Gln Leu Val Glu Ser Gly Gly Gly Val Val Gln Pro Gly Arg
1               5                   10                  15

Ser Leu Arg Leu Ser Cys Ala Ala Ser Gly Phe Thr Phe Thr Asn Tyr
            20                  25                  30

Gly Met His Trp Gly Arg Gln Ala Pro Gly Lys Gly Leu Glu Trp Val
        35                  40                  45

Ala Val Ile Ser His Asp Gly Asn Asn Lys Tyr Tyr Val Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Glu Gly Ile Asp Phe Trp Ser Gly Leu Asn Trp Phe Asp Pro
            100                 105                 110

Trp Gly Gln Gly Thr Leu Val Thr Val Ser Ser Ala
        115                 120

15
327
DNA
Homo sapiens

15
gaaattgtgt tgacgcagtc tccaggcacc ctgtctttgt ctccagggga aagagccact 60

ctctcctgca gggccagtca gagtattacc ggcagctact tagcctggta ccagcagaaa 120

cctggccagg ctcccagact cctcatctgt ggtgcatcca gctgggccac tggcatccca 180

gacaggttca gtggcagtgg gtctgggaca gacttcactc tcaccatcag tagactggag 240

cctgaagatt ttgcagtgta ttactgtcag cagtatagta gttcaccgat caccttcggc 300

caagggacac gactggagat taaacga 327

16
109
PRT
Homo sapiens

16
Glu Ile Val Leu Thr Gln Ser Pro Gly Thr Leu Ser Leu Ser Pro Gly
1               5                   10                  15

Glu Arg Ala Thr Leu Ser Cys Arg Ala Ser Gln Ser Ile Thr Gly Ser
            20                  25                  30

Tyr Leu Ala Trp Tyr Gln Gln Lys Pro Gly Gln Ala Pro Arg Leu Leu
        35                  40                  45

Ile Cys Gly Ala Ser Ser Trp Ala Thr Gly Ile Pro Asp Arg Phe Ser
    50                  55                  60

Gly Ser Gly Ser Gly Thr Asp Phe Thr Leu Thr Ile Ser Arg Leu Glu
65                  70                  75                  80

Pro Glu Asp Phe Ala Val Tyr Tyr Cys Gln Gln Tyr Ser Ser Ser Pro
                85                  90                  95

Ile Thr Phe Gly Gln Gly Thr Arg Leu Glu Ile Lys Arg
            100                 105

17
372
DNA
Homo sapiens

17
caggtgcagc tggtggagtc tgggggaggc gtggtccagc ctgggaggtc cctgagactc      60

tcctgtgcag cctctggatt caccttcact aactatggca tgcactgggg ccgccaggct     120

ccaggcaagg ggctggagtg ggtggcagtt atatcacatg atggaaataa taagtattat     180

gtagactccg tgaagggccg attcaccatc tccagagaca attccaagaa cacgctgtat     240

ctgcaaatga acagcctgag agctgaggac acggctgtgt attactgtgc gagagaggga     300

atcgattttt ggagtggcct caactggttc gacccctggg gccagggaac cctggtcacc     360

gtctcctcag cc                                                         372

18
124
PRT
Homo sapiens

18
Gln Val Gln Leu Val Glu Ser Gly Gly Gly Val Val Gln Pro Gly Arg
1               5                   10                  15

Ser Leu Arg Leu Ser Cys Ala Ala Ser Gly Phe Thr Phe Thr Asn Tyr
            20                  25                  30

Gly Met His Trp Gly Arg Gln Ala Pro Gly Lys Gly Leu Glu Trp Val
        35                  40                  45

Ala Val Ile Ser His Asp Gly Asn Asn Lys Tyr Tyr Val Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys

85                  90                  95

Ala Arg Glu Gly Ile Asp Phe Trp Ser Gly Leu Asn Trp Phe Asp Pro
            100                 105                 110

Trp Gly Gln Gly Thr Leu Val Thr Val Ser Ser Ala
        115                 120

19
327
DNA
Homo sapiens

19
gaaattgtgt tgacgcagtc tccaggcacc ctgtctttgt ctccagggga aagagccact      60

ctctcctgca gggccagtca gagtattacc ggcagctact tagcctggta ccagcagaaa     120

cctggccagg ctcccagact cctcatctgt ggtgcatcca gctgggccac tggcatccca     180

gacaggttca gtggcagtgg gtctgggaca gacttcactc tcaccatcag tagactggag     240

cctgaagatt ttgcagtgta ttactgtcag cagtatagta gttcaccgat caccttcggc     300

caagggacac gactggagat taaacga                                         327

20
109
PRT
Homo sapiens

20
Glu Ile Val Leu Thr Gln Ser Pro Gly Thr Leu Ser Leu Ser Pro Gly
1               5                   10                  15

Glu Arg Ala Thr Leu Ser Cys Arg Ala Ser Gln Ser Ile Thr Gly Ser
            20                  25                  30

Tyr Leu Ala Trp Tyr Gln Gln Lys Pro Gly Gln Ala Pro Arg Leu Leu
        35                  40                  45

Ile Cys Gly Ala Ser Ser Trp Ala Thr Gly Ile Pro Asp Arg Phe Ser
    50                  55                  60

Gly Ser Gly Ser Gly Thr Asp Phe Thr Leu Thr Ile Ser Arg Leu Glu
65                  70                  75                  80

Pro Glu Asp Phe Ala Val Tyr Tyr Cys Gln Gln Tyr Ser Ser Ser Pro
                85                  90                  95

Ile Thr Phe Gly Gln Gly Thr Arg Leu Glu Ile Lys Arg
            100                 105

21
363

DNA
Homo sapiens

21
gaggtgcaga tggtggagtc tgggggaggc ttggtccagc cgggggggtc cctgagactc 60

tcctgtgcag cctctggatt caccttaaga agctactgga tgagctgggt ccgccaggct 120

ccagggaagg ggctggagtg ggtggccaac ataaaggaag acggaagtga gaaataccat 180

gtggactctg tgaagggccg attcaccatc tccagagaca acgccgagaa ctcactgttt 240

ctgcaaatga gcagcctgcg agccgaggac acggctgtgt attactgtgc gagagatatg 300

gaagcatcag ctggcctctt tgactactgg ggccagggaa ccctggtcac cgtctcctca 360

gct 363

22
121
PRT
Homo sapiens

22
Glu Val Gln Met Val Glu Ser Gly Gly Gly Leu Val Gln Pro Gly Gly
1               5                   10                  15

Ser Leu Arg Leu Ser Cys Ala Ala Ser Gly Phe Thr Leu Arg Ser Tyr
            20                  25                  30

Trp Met Ser Trp Val Arg Gln Ala Pro Gly Lys Gly Leu Glu Trp Val
        35                  40                  45

Ala Asn Ile Lys Glu Asp Gly Ser Glu Lys Tyr His Val Asp Ser Val
    50                  55                  60

Lys Gly Arg Phe Thr Ile Ser Arg Asp Asn Ala Glu Asn Ser Leu Phe
65                  70                  75                  80

Leu Gln Met Ser Ser Leu Arg Ala Glu Asp Thr Ala Val Tyr Tyr Cys
                85                  90                  95

Ala Arg Asp Met Glu Ala Ser Ala Gly Leu Phe Asp Tyr Trp Gly Gln
            100                 105                 110

Gly Thr Leu Val Thr Val Ser Ser Ala
        115                 120

23
321
DNA
Homo sapiens

23
gaaatagtga tgacgcagtc cccagccacc ctgtctgtgt ctccagggga aagagccatc 60

ctctcctgca gggccagtca gagtattagc agcaacttag cctggtacca gcagaaacct 120

ggccaggctc ccaggctcct catctatggt gcatccacca gggccactgg tatcccagcc 180

aggttcagtg gcagtgggtc tgggacagag ttcactctca ccatcagcag cctgcagtct 240

gaagattttg cagtttatta ctgtcagcag tataattact ggtggacgtt cggccaaggg 300

accaaggtgg aaatcaaacg a 321

24
107
PRT
Homo sapiens

24
Glu Ile Val Met Thr Gln Ser Pro Ala Thr Leu Ser Val Ser Pro Gly
1               5                   10                  15

Glu Arg Ala Ile Leu Ser Cys Arg Ala Ser Gln Ser Ile Ser Ser Asn
            20                  25                  30

Leu Ala Trp Tyr Gln Gln Lys Pro Gly Gln Ala Pro Arg Leu Leu Ile
        35                  40                  45

Tyr Gly Ala Ser Thr Arg Ala Thr Gly Ile Pro Ala Arg Phe Ser Gly
    50                  55                  60

Ser Gly Ser Gly Thr Glu Phe Thr Leu Thr Ile Ser Ser Leu Gln Ser
65                  70                  75                  80

Glu Asp Phe Ala Val Tyr Tyr Cys Gln Gln Tyr Asn Tyr Trp Trp Thr
                85                  90                  95

Phe Gly Gln Gly Thr Lys Val Glu Ile Lys Arg
            100                 105

25
363
DNA
Homo sapiens

25
gaggtgcaga tggtggagtc tgggggaggc ttggtccagc cgggggggtc cctgagactc 60

tcctgtgcag cctctggatt caccttaaga agctactgga tgagctgggt ccgccaggct 120

ccagggaagg ggctggagtg ggtggccaac ataaaggaag acggaagtga gaaataccat 180

gtggactctg tgaagggccg attcaccatc tccagagaca acgccgagaa ctcactgttt 240

ctgcaaatga gcagcctgcg agccgaggac acggctgtgt attactgtgc gagagatatg 300

gaagcatcag ctggcctctt tgactactgg ggccagggaa ccctggtcac cgtctcctca 360

gct 363

26
121
PRT
Homo sapiens

26
Glu Val Gln Met Val Glu Ser Gly Gly Gly Leu Val Gln Pro Gly Gly
1               5                   10                  15

Ser Leu Arg Leu Ser Cys Ala Ala Ser Gly Phe Thr Leu Arg Ser Tyr
            20                  25                  30

Trp Met Ser Trp Val Arg Gln Ala Pro Gly Lys Gly Leu Glu Trp Val
        35                  40                  45

Ala Asn Ile Lys Glu Asp Gly Ser Glu Lys Tyr His Val Asp Ser Val
    50                  55                  60

Lys Gly Arg Phe Thr Ile Ser Arg Asp Asn Ala Glu Asn Ser Leu Phe
65                  70                  75                  80

Leu Gln Met Ser Ser Leu Arg Ala Glu Asp Thr Ala Val Tyr Tyr Cys
                85                  90                  95

Ala Arg Asp Met Glu Ala Ser Ala Gly Leu Phe Asp Tyr Trp Gly Gln
            100                 105                 110

Gly Thr Leu Val Thr Val Ser Ser Ala
        115                 120

27
321
DNA
Homo sapiens

27
gaaatagtga tgacgcagtc cccagccacc ctgtctgtgt ctccagggga aagagccatc        60

ctctcctgca gggccagtca gagtattagc agcaacttag cctggtacca gcagaaacct       120

ggccaggctc ccaggctcct catctatggt gcatccacca gggccactgg tatcccagcc       180

aggttcagtg gcagtgggtc tgggacagag ttcactctca ccatcagcag cctgcagtct       240

gaagattttg cagtttatta ctgtcagcag tataattact ggtggacgtt cggccaaggg       300

accaaggtgg aaatcaaacg a                                                 321

28
107
PRT
Homo sapiens

28
Glu Ile Val Met Thr Gln Ser Pro Ala Thr Leu Ser Val Ser Pro Gly
1               5                   10                  15

Glu Arg Ala Ile Leu Ser Cys Arg Ala Ser Gln Ser Ile Ser Ser Asn
            20                  25                  30

Leu Ala Trp Tyr Gln Gln Lys Pro Gly Gln Ala Pro Arg Leu Leu Ile
        35                  40                  45

Tyr Gly Ala Ser Thr Arg Ala Thr Gly Ile Pro Ala Arg Phe Ser Gly
    50                  55                  60

Ser Gly Ser Gly Thr Glu Phe Thr Leu Thr Ile Ser Ser Leu Gln Ser
65                  70                  75                  80

Glu Asp Phe Ala Val Tyr Tyr Cys Gln Gln Tyr Asn Tyr Trp Trp Thr
                85                  90                  95

Phe Gly Gln Gly Thr Lys Val Glu Ile Lys Arg
            100                 105

29
363
DNA
Homo sapiens

29
gaggtgcaga tggtggagtc tgggggaggc ttggtccagc cgggggggtc cctgagactc 60

tcctgtgcag cctctggatt caccttaaga agctactgga tgagctgggt ccgccaggct 120

ccagggaagg ggctggagtg ggtggccaac ataaaggaag acggaagtga gaaataccat 180

gtggactctg tgaagggccg attcaccatc tccagagaca acgccgagaa ctcactgttt 240

ctgcaaatga gcagcctgcg agccgaggac acggctgtgt attactgtgc gagagatatg 300

gaagcatcag ctggcctctt tgactactgg ggccagggaa ccctggtcac cgtctcctca 360

gct 363

30
121
PRT
Homo sapiens

30
Glu Val Gln Met Val Glu Ser Gly Gly Gly Leu Val Gln Pro Gly Gly
1               5                   10                  15

Ser Leu Arg Leu Ser Cys Ala Ala Ser Gly Phe Thr Leu Arg Ser Tyr
            20                  25                  30

Trp Met Ser Trp Val Arg Gln Ala Pro Gly Lys Gly Leu Glu Trp Val
        35                  40                  45

Ala Asn Ile Lys Glu Asp Gly Ser Glu Lys Tyr His Val Asp Ser Val
    50                  55                  60

Lys Gly Arg Phe Thr Ile Ser Arg Asp Asn Ala Glu Asn Ser Leu Phe
65                  70                  75                  80

Leu Gln Met Ser Ser Leu Arg Ala Glu Asp Thr Ala Val Tyr Tyr Cys
                85                  90                  95

Ala Arg Asp Met Glu Ala Ser Ala Gly Leu Phe Asp Tyr Trp Gly Gln
            100                 105                 110

Gly Thr Leu Val Thr Val Ser Ser Ala
        115                 120

31
321
DNA
Homo sapiens

31
gaaatagtga tgacgcagtc cccagccacc ctgtctgtgt ctccagggga aagagccatc 60

ctctcctgca gggccagtca gagtattagc agcaacttag cctggtacca gcagaaacct 120

ggccaggctc ccaggctcct catctatggt gcatccacca gggccactgg tatcccagcc 180

aggttcagtg gcagtgggtc tgggacagag ttcactctca ccatcagcag cctgcagtct 240

gaagattttg cagtttatta ctgtcagcag tataattact ggtggacgtt cggccaaggg 300

accaaggtgg aaatcaaacg a 321

32
107
PRT
Homo sapiens

32
Glu Ile Val Met Thr Gln Ser Pro Ala Thr Leu Ser Val Ser Pro Gly
1               5                   10                  15

Glu Arg Ala Ile Leu Ser Cys Arg Ala Ser Gln Ser Ile Ser Ser Asn
            20                  25                  30

Leu Ala Trp Tyr Gln Gln Lys Pro Gly Gln Ala Pro Arg Leu Leu Ile
        35                  40                  45

Tyr Gly Ala Ser Thr Arg Ala Thr Gly Ile Pro Ala Arg Phe Ser Gly
    50                  55                  60

Ser Gly Ser Gly Thr Glu Phe Thr Leu Thr Ile Ser Ser Leu Gln Ser
65                  70                  75                  80

Glu Asp Phe Ala Val Tyr Tyr Cys Gln Gln Tyr Asn Tyr Trp Trp Thr
                85                  90                  95

Phe Gly Gln Gly Thr Lys Val Glu Ile Lys Arg
            100                 105

 33
 215
 PRT
 Artificial Sequence

 Description of Artificial Sequence: Synthetic
      polypeptide

 33
Glu Ile Val Leu Thr Gln Ser Pro Gly Thr Leu Ser Leu Ser Pro Gly
1               5                   10                  15

Glu Arg Ala Thr Leu Ser Cys Arg Ala Ser Gln Ser Ile Thr Gly Ser
            20                  25                  30

Tyr Leu Ala Trp Tyr Gln Gln Lys Pro Gly Gln Ala Pro Arg Leu Leu
        35                  40                  45

Ile Thr Gly Ala Ser Ser Trp Ala Thr Gly Ile Pro Asp Arg Phe Ser
    50                  55                  60

Gly Ser Gly Ser Gly Thr Asp Phe Thr Leu Thr Ile Ser Arg Leu Glu
65                  70                  75                  80

Pro Glu Asp Phe Ala Val Tyr Tyr Cys Gln Gln Tyr Ser Ser Ser Pro
                85                  90                  95

Ile Thr Phe Gly Gln Gly Thr Arg Leu Glu Ile Lys Arg Thr Val Ala
            100                 105                 110

Ala Pro Ser Val Phe Ile Phe Pro Pro Ser Asp Glu Gln Leu Lys Ser
        115                 120                 125

Gly Thr Ala Ser Val Val Cys Leu Leu Asn Asn Phe Tyr Pro Arg Glu
    130                 135                 140

Ala Lys Val Gln Trp Lys Val Asp Asn Ala Leu Gln Ser Gly Asn Ser
145                 150                 155                 160

Gln Glu Ser Val Thr Glu Gln Asp Ser Lys Asp Ser Thr Tyr Ser Leu
                165                 170                 175

Ser Ser Thr Leu Thr Leu Ser Lys Ala Asp Tyr Glu Lys His Lys Val
            180                 185                 190

Tyr Ala Cys Glu Val Thr His Gln Gly Leu Ser Ser Pro Val Thr Lys
        195                 200                 205

Ser Phe Asn Arg Gly Glu Cys
    210                 215

34
215
PRT
Artificial Sequence

Description of Artificial Sequence: Synthetic
polypeptide

34
Glu Ile Val Leu Thr Gln Ser Pro Gly Thr Leu Ser Leu Ser Pro Gly
1               5                   10                  15

Glu Arg Ala Thr Leu Ser Cys Arg Ala Ser Gln Ser Ile Thr Gly Ser
            20                  25                  30

Tyr Leu Ala Trp Tyr Gln Gln Lys Pro Gly Gln Ala Pro Arg Leu Leu
        35                  40                  45

Ile Asn Gly Ala Ser Ser Trp Ala Thr Gly Ile Pro Asp Arg Phe Ser
    50                  55                  60

Gly Ser Gly Ser Gly Thr Asp Phe Thr Leu Thr Ile Ser Arg Leu Glu
65                  70                  75                  80

Pro Glu Asp Phe Ala Val Tyr Tyr Cys Gln Gln Tyr Ser Ser Ser Pro
                85                  90                  95

Ile Thr Phe Gly Gln Gly Thr Arg Leu Glu Ile Lys Arg Thr Val Ala
            100                 105                 110

Ala Pro Ser Val Phe Ile Phe Pro Pro Ser Asp Glu Gln Leu Lys Ser
        115                 120                 125

Gly Thr Ala Ser Val Val Cys Leu Leu Asn Asn Phe Tyr Pro Arg Glu
    130                 135                 140

Ala Lys Val Gln Trp Lys Val Asp Asn Ala Leu Gln Ser Gly Asn Ser
145                 150                 155                 160

Gln Glu Ser Val Thr Glu Gln Asp Ser Lys Asp Ser Thr Tyr Ser Leu
                165                 170                 175

Ser Ser Thr Leu Thr Leu Ser Lys Ala Asp Tyr Glu Lys His Lys Val
        180                 185                 190

Tyr Ala Cys Glu Val Thr His Gln Gly Leu Ser Ser Pro Val Thr Lys
    195                 200                 205

Ser Phe Asn Arg Gly Glu Cys
    210                 215

35
215
PRT
Artificial Sequence

Description of Artificial Sequence: Synthetic
      polypeptide

35
Glu Ile Val Leu Thr Gln Ser Pro Gly Thr Leu Ser Leu Ser Pro Gly
1               5                   10                  15

Glu Arg Ala Thr Leu Ser Cys Arg Ala Ser Gln Ser Ile Thr Gly Ser
            20                  25                  30

Tyr Leu Ala Trp Tyr Gln Gln Lys Pro Gly Gln Ala Pro Arg Leu Leu
        35                  40                  45

Ile Asp Gly Ala Ser Ser Trp Ala Thr Gly Ile Pro Asp Arg Phe Ser
    50                  55                  60

Gly Ser Gly Ser Gly Thr Asp Phe Thr Leu Thr Ile Ser Arg Leu Glu
65                  70                  75                  80

Pro Glu Asp Phe Ala Val Tyr Tyr Cys Gln Gln Tyr Ser Ser Ser Pro
                85                  90                  95

Ile Thr Phe Gly Gln Gly Thr Arg Leu Glu Ile Lys Arg Thr Val Ala
            100                 105                 110

Ala Pro Ser Val Phe Ile Phe Pro Pro Ser Asp Glu Gln Leu Lys Ser
        115                 120                 125

Gly Thr Ala Ser Val Val Cys Leu Leu Asn Asn Phe Tyr Pro Arg Glu
    130                 135                 140

Ala Lys Val Gln Trp Lys Val Asp Asn Ala Leu Gln Ser Gly Asn Ser
145                 150                 155                 160

Gln Glu Ser Val Thr Glu Gln Asp Ser Lys Asp Ser Thr Tyr Ser Leu
                165                 170                 175

Ser Ser Thr Leu Thr Leu Ser Lys Ala Asp Tyr Glu Lys His Lys Val
            180                 185                 190

Tyr Ala Cys Glu Val Thr His Gln Gly Leu Ser Ser Pro Val Thr Lys
        195                 200                 205

Ser Phe Asn Arg Gly Glu Cys
    210                 215

36
215
PRT
Artificial Sequence

Description of Artificial Sequence: Synthetic
polypeptide

36
Glu Ile Val Leu Thr Gln Ser Pro Gly Thr Leu Ser Leu Ser Pro Gly
1               5                   10                  15

Glu Arg Ala Thr Leu Ser Cys Arg Ala Ser Gln Ser Ile Thr Gly Ser
            20                  25                  30

Tyr Leu Ala Trp Tyr Gln Gln Lys Pro Gly Gln Ala Pro Arg Leu Leu
        35                  40                  45

Ile Ala Gly Ala Ser Ser Trp Ala Thr Gly Ile Pro Asp Arg Phe Ser
    50                  55                  60

Gly Ser Gly Ser Gly Thr Asp Phe Thr Leu Thr Ile Ser Arg Leu Glu
65                  70                  75                  80

Pro Glu Asp Phe Ala Val Tyr Tyr Cys Gln Gln Tyr Ser Ser Ser Pro
                85                  90                  95

Ile Thr Phe Gly Gln Gly Thr Arg Leu Glu Ile Lys Arg Thr Val Ala
            100                 105                 110

Ala Pro Ser Val Phe Ile Phe Pro Pro Ser Asp Glu Gln Leu Lys Ser
        115                 120                 125

Gly Thr Ala Ser Val Val Cys Leu Leu Asn Asn Phe Tyr Pro Arg Glu
    130                 135                 140

Ala Lys Val Gln Trp Lys Val Asp Asn Ala Leu Gln Ser Gly Asn Ser
145                 150                 155                 160

Gln Glu Ser Val Thr Glu Gln Asp Ser Lys Asp Ser Thr Tyr Ser Leu
                165                 170                 175

Ser Ser Thr Leu Thr Leu Ser Lys Ala Asp Tyr Glu Lys His Lys Val
            180                 185                 190

Tyr Ala Cys Glu Val Thr His Gln Gly Leu Ser Ser Pro Val Thr Lys
        195                 200                 205

Ser Phe Asn Arg Gly Glu Cys
    210                 215

37
123
PRT
Artificial Sequence

Description of Artificial Sequence: Synthetic
     polypeptide

37
Gln Val Gln Leu Val Glu Ser Gly Gly Gly Val Val Gln Pro Gly Arg
1               5                   10                  15

Ser Leu Arg Leu Ser Cys Ala Ala Ser Gly Phe Thr Phe Thr Asn Tyr
            20                  25                  30

Gly Met His Trp Val Arg Gln Ala Pro Gly Lys Gly Leu Glu Trp Val
        35                  40                  45

Ala Val Ile Ser His Asp Gly Asn Asn Lys Tyr Tyr Val Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Glu Gly Ile Asp Phe Trp Ser Gly Leu Asn Trp Phe Asp Pro
            100                 105                 110

Trp Gly Gln Gly Thr Leu Val Thr Val Ser Ser
        115                 120

38
215
PRT
Artificial Sequence

Description of Artificial Sequence: Synthetic

polypeptide

38
Glu Ile Val Leu Thr Gln Ser Pro Gly Thr Leu Ser Leu Ser Pro Gly
1               5                   10                  15

Glu Arg Ala Thr Leu Ser Cys Arg Ala Ser Gln Ser Ile Thr Gly Ser
            20                  25                  30

Tyr Leu Ala Trp Tyr Gln Gln Lys Pro Gly Gln Ala Pro Arg Leu Leu
        35                  40                  45

Ile Thr Gly Ala Ser Ser Trp Ala Thr Gly Ile Ala Asp Arg Phe Ser
    50                  55                  60

Gly Ser Gly Ser Gly Thr Asp Phe Thr Leu Thr Ile Ser Arg Leu Glu
65                  70                  75                  80

Pro Glu Asp Phe Ala Val Tyr Tyr Cys Gln Gln Tyr Ser Ser Ser Pro
                85                  90                  95

Ile Thr Phe Gly Gln Gly Thr Arg Leu Glu Ile Lys Arg Thr Val Ala
            100                 105                 110

Ala Pro Ser Val Phe Ile Phe Pro Pro Ser Asp Glu Gln Leu Lys Ser
        115                 120                 125

Gly Thr Ala Ser Val Val Cys Leu Leu Asn Asn Phe Tyr Pro Arg Glu
    130                 135                 140

Ala Lys Val Gln Trp Lys Val Asp Asn Ala Leu Gln Ser Gly Asn Ser
145                 150                 155                 160

Gln Glu Ser Val Thr Glu Gln Asp Ser Lys Asp Ser Thr Tyr Ser Leu
                165                 170                 175

Ser Ser Thr Leu Thr Leu Ser Lys Ala Asp Tyr Glu Lys His Lys Val
            180                 185                 190

Tyr Ala Cys Glu Val Thr His Gln Gly Leu Ser Ser Pro Val Thr Lys
        195                 200                 205

Ser Phe Asn Arg Gly Glu Cys
 210            215

## Claims

1. An article of manufacture comprising:
one or more non-glass primary packaging components comprising one or more containers containing a liquid antibody formulation having an improved aggregation profile, the liquid formulation comprising a fully human monoclonal antibody that binds to angiopoietin-2 in a pharmaceutically acceptable carrier;
wherein no more than about 5% of the antibody in the formulation forms an aggregate.

2. The article of manufacture of claim 1, wherein one or more containers are non-glass containers selected from an ampule, vial, bottle and combinations thereof and wherein the non-glass container comprises polymer selected from polypropylene (PP), polyethylene terephthalate (PETG), high-density polyethylene (HDPE), polyethylene terephthalate (PET), polypentafluorostyrene (PFS), polycarbonate, polyvinyl chloride (PVC), polycyclopentane (CZ®), cyclic olefin copolymer (COC), and combinations or copolymers thereof.

3. The article of manufactured of claim 2, wherein the non-glass container comprises polypropylene.

4. The article of manufacture of any one of claims 1 - 3, wherein the container contains air and the liquid formulation in a ratio of volumeₐᵢᵣ to volume_{liquid} ratios of less than or equal to 0.05.

5. The article of manufacture of any one of claims 1 - 4, wherein at least one primary packaging component comprises a container closure selected from polypropylene (PP), polyethylene terephthalate (PETG), high-density polyethylene (HDPE), polyethylene terephthalate (PET), polypentafluorostyrene (PFS), polycarbonate, polyvinyl chloride (PVC), polycyclopentane (CZ®), cyclic olefin copolymer (COC), and combinations or copolymers thereof.

6. The article of manufacture of any one of claims 1 - 5, wherein the fully human monoclonal antibody that binds to angiopoietin-2 is selected from mAb 3.19.3 (ATCC Accession Number PTA-7260); mAb 3.3.2 (ATCC Accession Number PTA-7258); and mAb 5.88.3 (ATCC Accession Number PTA-7259).

7. The article of manufacture of any one of claims 1 - 6, wherein the fully human monoclonal antibody comprises a variable light chain and a variable heavy chain member selected from:
a light chain sequence comprising SEQ ID NO: 12 and a heavy chain sequence comprising SEQ ID NO: 10;
a light chain sequence comprising SEQ ID NO: 20 and a heavy chain sequence comprising SEQ ID NO: 18; and
a light chain sequence comprising SEQ ID NO: 32 and a heavy chain sequence comprising SEQ ID NO: 30.

8. The article of manufacture of any one of claims 1 - 6, wherein the fully human monoclonal antibody is selected from an antibody comprising heavy chain CDR1, CDR2, CDR3 of SEQ ID NO: 10 and light chain CDR1, CDR2, CDR3 of SEQ ID NO: 12; heavy chain CDR1, CDR2, CDR3 of SEQ ID NO: 18 and light chain CDR1, CDR2, CDR3 of SEQ ID NO: 20; and heavy chain CDR1, CDR2, CDR3 of SEQ ID NO: 30 and light chain CDR1, CDR2, CDR3 of SEQ ID NO: 32.

9. The article of manufacture of any one of claims 1 - 6, wherein the fully human monoclonal antibody comprises a variable light chain comprising a sequence selected from SEQ ID No:33 (MEDI1); SEQ ID No:34 (MEDI2); SEQ ID No:35 (MEDI3); SEQ ID No:36 (MEDI4); and SEQ ID No:38 (MEDI6) and wherein the fully human monoclonal antibody further comprises a variable heavy chain region comprising SEQ ID No:37 (MEDI5).

10. The article of manufacture of any one of claims 1 - 9, wherein the liquid formulation has a pH between about 5.0 and about 8.0.

11. The article of manufacture of any one of claims 1 - 10, wherein the antibody aggregate comprises visible particles, subvisible particles, precipitates, fibrils, or a combination thereof.

12. The article of manufacture of claim 11, wherein the aggregate comprises sub-visible particles greater than at least about 2 µm and up to about 200 µm.

13. The article of manufacture of any one of claims 1 - 12, comprising at least about 0% and up to about 1% of a surfactant and wherein the surfactant is a nonionic surfactant selected from polysorbate, poloxamer and combinations thereof.

14. The article of manufacture of claim 13, wherein polysorbate is selected from polysorbate 20 (PS-20), polysorbate 80, and combinations thereof.

15. The article of manufacture of claim 14, wherein the polysorbate is PS-20.

16. The article of manufacture of claim 1, wherein the liquid antibody formulation comprises at least one monoclonal antibody in between about 0.01% and 0.05% polysorbate-20, at a pH of between about 5.0 and 8.0.
